# EUROPEAN PATENT SPECIFICATION

(11) **EP 0 965 041 B1**
(45) Date of publication and mention of the grant of the patent: **18.02.2009**
(21) Application number: 98918196.1
(22) Date of filing: 14.04.1998
(51) Int. Cl.: G01N 33/50, C07K 14/00, C07K 14/72, G01N 33/566, C07K 14/705

(54) **A METHOD OF IDENTIFYING MODULATORS OF CELL SURFACE MEMBRANE RECEPTORS USEFUL IN THE TREATMENT OF DISEASE**
METHODE ZUM IDENTIFIZIEREN VON MODULATOREN DER ZELLOBERFLÄCHENMEMBRANREZEPTOREN,NÜTZLICH ZUR BEHANDLUNG VON KRANKHEITEN
PROCEDE POUR IDENTIFIER LES MODULATEURS DES RECEPTEURS MEMBRANAIRES DE SURFACES CELLULAIRES UTILES DANS LE TRAITEMENT DE MALADIES

(30) Priority: 14.04.1997 US 839449
(43) Date of publication of application: 22.12.1999
(73) Proprietor: Arena Pharmaceuticals, Inc., San Diego, CA 92121 (US)
(72) Inventor: Dominic P. Behan, San Diego, CA 92131 (US); Derek T. Chalmers, Connecticut 06878 (US)
(74) Representative: Cripps, Joanna Elizabeth
(86) International application number: PCT/US1998/007496
(87) International publication number: WO 1998/046995

(56) References cited:
- WO-A-97/11159
- WO-A-97/21731
- US-A- 5 514 578
- US-A- 5 532 157
- US-A- 5 532 157
- US-A- 5 573 944
- US-A- 5 639 616
- EGGERICKX D. et al., "Molecular Cloning of an Orphan G-Protein-Coupled Receptor that Constitutively Activates Adenylate Cyclase", BIOCHEMICAL JOURNAL, 1995, Vol. 309, pages 837-843, XP002912497
- OFFERMANNS S. et al., "Galpha 15 and Galpha 16 Couple a Wide Variety of Receptors to Phospholipase C", J. BIOL. CHEM., 23 June 1995, Vol. 270, No. 25, pages 15175-15180, XP002912498
- ZHANG S. et al., "Identification of Dynorphins as Endogenous Ligands for an Opioid Receptor-Like Orphan Receptor", J. BIOL. CHEM., 29 September 1995, Vol. 270, No. 39, pages 22772-22776, XP002912499
- CHEN J. et al., "Tethered Ligand Library for Discovery of Peptide Agonists", J. BIOL. CHEM., 06 October 1995, Vol. 270, No. 40, pages 23398-23401, XP002912500

## Description

### BACKGROUND OF THE INVENTION

### Field of the Invention

The present invention deals with intercellular communication in multicellular organisms and the regulation of cellular function and, more specifically, with regulation of membrane receptors which are constitutively activated as a result of genetic mutation, post-translational modification, or some other process.

### Description of the Related Art

### Cell Surface Receptors

Cells do not exist in isolation. Especially in multicellular organisms with differentiated tissues, communication between cells is necessary to coordinate the integration and functioning of the entire organism. Generally, this communication must take place over considerable distances. The information transfer function between cells must meet two general criteria for any information system: 1) the information must be capable of reaching its intended destination unchanged; and 2) the information should be delivered to and detected by only the recipient which will act upon the information. Failure of either of these two conditions results in meaningless noise being introduced into the system. As early as 1905, Langley introduced the concept that cells communicate by synthesizing and releasing chemical compound signals, which are detected by molecules located on the receiving cells which are especially designed to respond just to those signals.

In animals, three signal types are distinguished based upon the distances involved between signalling and receiving cells. To reach a distant group of cells, endocrine signalling involves the release by endocrine organs of hormones, usually into the bloodstream. The immune system also produces antibodies which may serve as signals for other cells located some distance away. For cases where the target cell is closer to the signalling cell, paracrine signalling involves signalling substances which affect only adjacent cells, such as in neurotransmission or neuromuscular transmission. Finally, some cells respond to compounds that they release. This self signalling is termed autocrine signalling.

Despite the variety of signal molecules employed by multicellular organisms (hormones, ions, neurotransmitters, immune molecules), the cellular signal receiving apparatus which has evolved for each is remarkably similar both in construction and operation. Each signalling molecule is recognized by a specific receptor consisting of a large macromolecular protein or an assembly of proteins embedded in and spanning the receiving cell plasma membrane. The part of the receptor which recognizes the signalling molecule lies on or above the extracellular surface of the cell. The parts of the protein receptor embedded in the membrane generally consist of amino acids with hydrophobic side groups. Finally, parts of the receptor extend into the intracellular space where they have access to cell chemistry.

The receptor acts to receive a specific extracellular signal and transmit its presence across the cell membrane to, activate an intracellular biochemical response. Exactly how this information is transferred across the cell membrane is not yet clearly understood. However, based on accumulating evidence, it is believed that the signal molecule stabilizes the receptor in a different three-dimensional shape (conformation), which shape difference in turn causes the intracellular parts to interact with a transducing protein which protein then participates in further intracellular biochemical activity. This further activity is designated a "second messenger" system and typically includes 3'5'-cyclic AMP (CAMP), 3'5'-cyclic GMP (CGMP), 1,2-diacylglycerol, inositol 1,4,5-triphosphate, and Ca²⁺. These second messengers in turn activate one or more enzymatic systems.

The exact specifics of this intracellular interaction vary depending upon the type of receptor. The same receptor occurring on different cells may be linked to different transducing proteins to produce different effects. Correspondingly, different receptors on the same cell may produce the same cellular response. Due to the fact that signalling substances bind to particular sites on the receptors to induce a conformational change, they are generally referred to as ligands.

A number of receptor classes are known to exist in mammals and these are differentiated on the basis of their genetic and chemical structure and the second messenger pathways with which they interact. There are four main receptor groups: 1) G protein-coupled receptors; 2) tyrosine kinase receptors; 3) ligand-gated ion channels; and 4) immune recognition receptors. These groups may also be differentiated into two classes based on the rapidity of their response when exposed to their appropriate ligand. The ligand-gated ion channels have millisecond responses when activated. Receptors belonging to the other groups mediate slower responses since additional second messenger pathways must be activated.

The traditional biochemical method of investigating receptors has and still follows the path of first trying to identify the endogenous or native ligand which activates the receptor. Sometimes the endogenous ligand has been known or was easily deduced such as in the case of neurotransmitters. Other times, based upon the physiological distribution of the receptor in various tissues, educated guesses have led to the correct ligand. Whatever the mode of discovery, all previously published approaches to studying receptors up to the present rely exclusively, with one exception, on the initial identification of the endogenous ligand. The sole exception is the instance when a compound is known to bind to a receptor, such as morphine, and is labelled with a radioisotope and then used to study the receptor. This can be done without the endogenous ligand, e.g., the enkephalin, endorphin, or dynorphin peptides for the opiate receptors. In this case, the radioactive compound may be used to identify the receptor and to help in the discovery and identification of the endogenous ligand(s).

Once the endogenous ligand has been identified, a search for compounds (drugs) which can be used to investigate or modify the behavior of the receptor are typically conducted. These searches, chemical library screening methods for identifying drug candidates at G protein-coupled (and other types of receptors), rely on ligand-based assays in which the ligand is first labelled in some manner (e.g., with a radioisotope) to allow detection and then used to "bind" the target receptor in an *in vitro* expression system. Chemical compounds are then screened across the target receptor in order to identify molecules which displace the labelled ligand from the receptor site. Molecules which displace the labelled ligand are potential agonists or antagonists (i.e., receptor stimulators or inhibitors) and serve as the chemical starting point for drug discovery. Listed in Appendix "A" are examples of drugs and other non-peptide compounds which have been discovered using ligand-based receptor screening assays. So well established is the ligand-based screening assay paradigm that major commercial enterprises have been built upon providing screening services. An example of the extensive repertoire of assays available from just one company is shown in Appendix "B".

However, to date, despite intense efforts, identification of endogenous ligands for novel receptors identified by molecular cloning has proven to be a very time consuming and, for the most part, unsuccessful endeavor. These receptors for which an endogenous ligand remains unknown are generically classified as "orphan receptors". By definition, the orphan receptor has no known ligand, either endogenous or synthetic, which can be used to study the receptor. For this reason, development of ligand-based screening assays and subsequent screening of chemical libraries against these receptors has not been possible. This has severely hampered small molecule drug discovery for this important class of receptors. As the Human Genome Project progresses, it is expected that in the near future the genetic sequences of additional receptors will be identified, and that such knowledge will lead to discovery of additional receptors responsible for human disease. Further, it is most likely that these receptors will also be classified as orphan receptors even if a direct linkage to a disease state is shown, since there is unlikely to be anything known about the endogenous ligands for these receptors. Clearly, it is critically important to find some method of identifying compounds which will modify the state of such receptors (to active or inactive depending on the circumstances of the disease) even in absence of knowledge of the endogenous ligand. However, no such method is known in the prior art.

### G protein-coupled Receptors

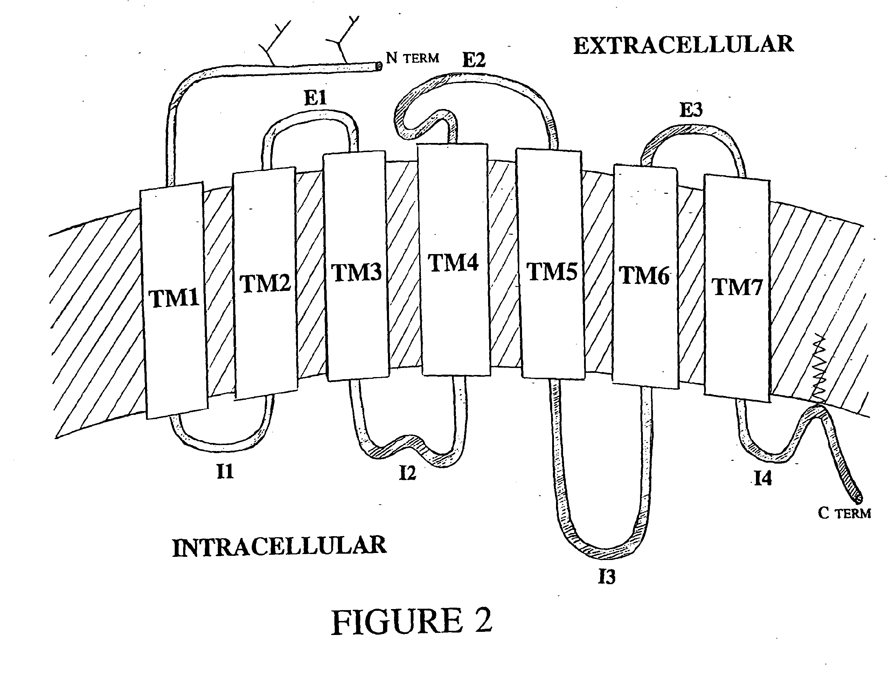

Of the four receptor groups mentioned above, the G protein-coupled receptors are the most numerous. G protein-coupled receptors are distinguished by the fact that the intracellular protein segment which the receptor activates binds GDP (guanosine diphosphate). After the G protein is activated by the receptor, bound GDP is converted to GTP (guanosine triphosphate) and the G protein mediates further enzymatic activity. Different G proteins mediate different intracellular activities. Despite the differences in their activating ligand, secondary messenger G protein systems and amino acid sequences, G protein-coupled receptors share a common structural motif. All these receptors have seven sequences of between 22 to 24 hydrophobic amino acids which form seven alpha ("α") helixes, each of which spans the membrane. The transmembrane helixes are joined by strands of amino acids with a larger loop between the fourth and fifth transmembrane helix on the extracellular side of the membrane. Another larger loop composed primarily of hydrophilic amino acids joins transmembrane helixes five and six on the intracellular side of the membrane. The carboxy terminus of the receptor lies intracellularly with the amino terminus in the extracellular space. It is thought that the loop joining helixes five and six as well as the carboxy terminus interact with the G protein. The general structure of G protein-coupled receptors is shown in Figure 2.

Within the human genome, an estimated total of 2000 genes are believed to code for this one class of receptor. To date, the genetic sequences for only about 100 G protein-coupled receptors for which the activating ligand is known have been identified. However, sequences for about 100 other G protein-coupled receptors for which the activating ligand is unknown have been identified and cloned. These represent the orphan G protein-coupled receptors.

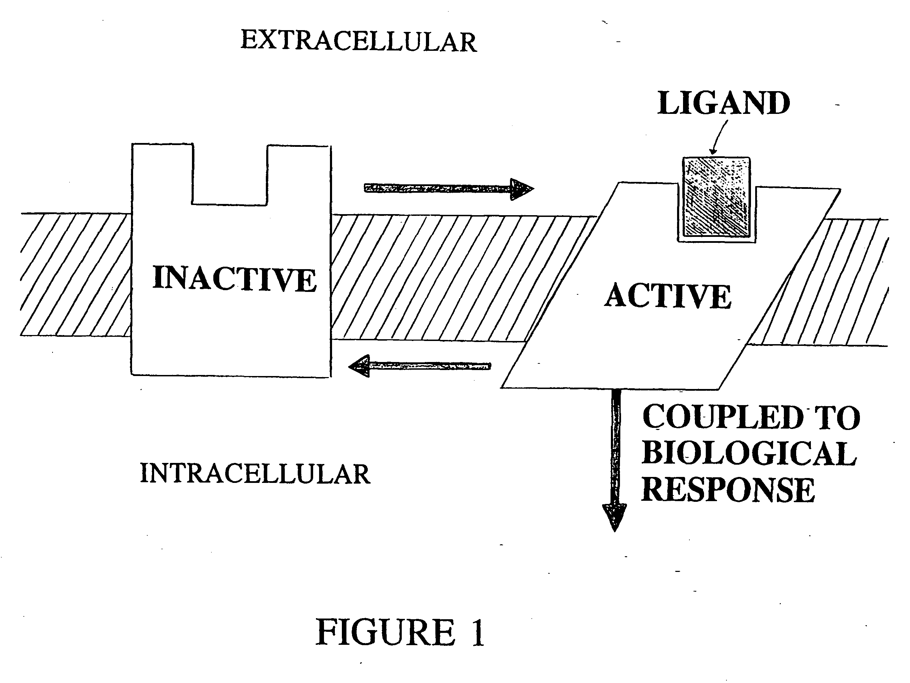

Under physiological conditions, G protein-coupled receptors exist in the cell membrane in equilibrium between two different states or conformations: an "inactive" state and an "active" state. As is shown schematically in Figure 1, in the inactive state the receptor is unable to link to the intracellular transduction (second messenger) pathway and no biological response is produced. A change of receptor conformation to the active state allows linkage to the transduction pathway and results in a biological response in the cell. Agonist ligands specific to the receptor are able to produce a biological response by stabilizing the receptor in the active state, thereby promoting linkage to the transduction pathway.

As will be discussed below in greater detail, recent discoveries over the past several years have shown that receptors can also be stabilized in the active confirmation by means other than binding with the endogenous ligand or an exogenous agonist ligand. Such other means include, but are not exclusively limited to, modifications to the amino acid sequence of the receptor. These other means effectively (in the absence of ligand) simulate the stabilizing effect of ligand binding to the receptor to keep the receptor in the active state. Stabilization by such ligand-independent means is termed "constitutive receptor activation". For instance, it was shown that interchange of a short homologous sequence of amino acids in the carboxy terminal of the third cytoplasmic loop of the G_{q}-phospholipase G protein coupled α_{1β} adrenoceptor and the Gₛ-adenyl cyclase-coupled β₂ adrenergic receptor resulted in both receptors promoting intracellular activation levels comparable to the fully agonist-stimulated native receptors. Equally important, the constitutively activated receptors had an affinity for the normal agonists much greater than that of the native receptors at the same time that their affinity for antagonists was not increased. In fact, when several known agonists of varying activity (with respect to the native receptor) were examined, it was found that the greater the initial activity of the agonist, the greater was the increase in its affinity for the constitutively activated receptor.

Four principal methods have been identified which result in constitutive activation of receptors. The first involves molecular alterations in the receptor amino acid sequence at specific locations. These changes induce a conformational change in the receptor resulting in constitutive activation. The second method involves stimulation of the receptor with anti-peptide antibodies to the part of the receptor extending into the extracellular space. The antibodies seem to functionally simulate ligand binding and induce constitutive activation. The third method involves over-expression of the receptor in an in-vitro system. In such a system, many more receptors are in the active state at any one time. Finally, the fourth method involves over-expression of the G protein(s) responsible for coupling to the active state of the receptor. The presence of a higher concentration of transducing G proteins works to shift the receptor equilibrium towards the active state.

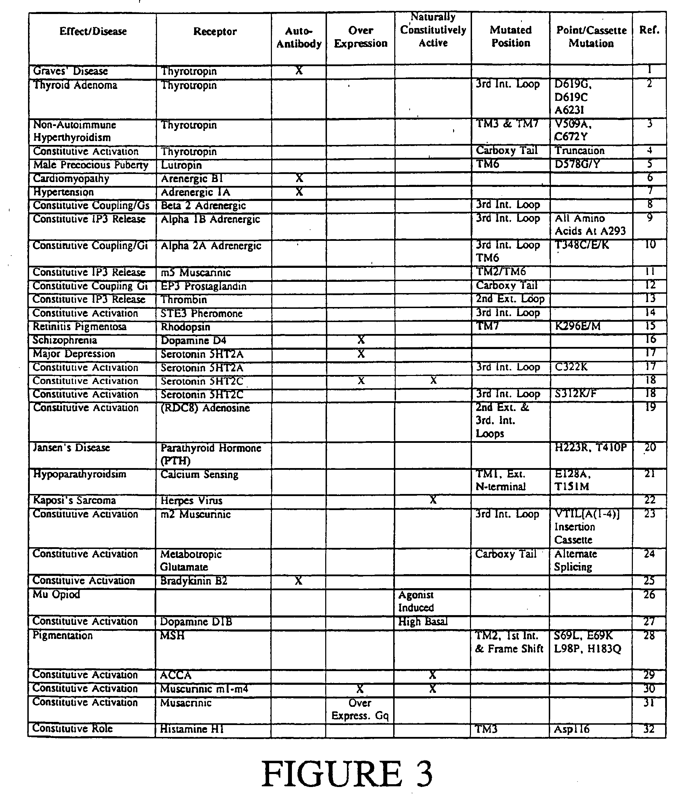

The importance of understanding constitutive activation of receptors has been further highlighted by the recent discoveries that a significant number of human diseases and serious medical conditions are related to constitutively activated receptors occurring naturally as a result of genetic mutations. In addition to those constitutively activated receptors associated with disease, several additional constitutively activated receptors are known which are not associated with disease. The chart in Figure 3 lists some of the known constitutively activated receptors and shows: 1) any associated disease; 2) the nature of the constitutive activation (sequence modification, antibody, overexpression); 3) the location of the activating modification; 4) the nature of the activating modification; and 5) reference citations. Literature references for each receptor shown in Figure 3 are provided in Appendix "C".

A summary of the constitutively activated receptors shown in Figure 3 and the molecular changes which have been identified in the receptors clearly demonstrates some of the basic requirements for constitutive activation. As set out below, some of these forms of constitutive activation occur naturally in disease states, while other forms are observed only in the laboratory.

Hyperthyroidism (Graves' disease): Graves' disease is known to be related to constitutive activation of the thyrotropin stimulating hormone (TSH) receptor in the thyroid gland, resulting in greatly increased circulating levels of thyroid hormones which leads to thyrotoxicosis. In most cases, this constitutive activity results from the stimulating action of naturally occurring autoantibodies against the thyrotropin receptor but, in some cases, a naturally occurring genetic alteration in the receptor can also cause constitutive activation (Parma et al. 1993; Duprez et al. 1994). Traditional treatment for Graves' disease has relied on either destruction of the thyroid gland, utilizing surgical ablation or radioiodine, or inhibition of thyroid hormone synthesis using anti-thyroid drugs (principally methimazole). Both treatments have proven to be less than ideal; after surgical or radioiodine treatments patients require continual thyroid hormone replacement therapy, while treatment with anti-thyroid drugs is associated with a relapse rate of 50-70% after 1 or 2 years treatment.

Male Precocious Puberty: This is usually an autosomal dominantly inherited disorder but it may also occur sporadically (Kosugi et al. 1995). Affected males show signs of virilization, often by age four, leading to eventual short stature. Low serum gonadotropins implicated gonadal hyperfunctioning in this disease, suggesting an overactivity of the luteinizing hormone (LH) receptor. Genetic analysis of affected members of several kindreds has revealed that natural mutations of aspartate 578 to glycine or tyrosine produces a constitutively active LH receptor. Interestingly, the tyrosine mutation was identified in a patient with precocious puberty by age one and was found to exhibit greater constitutive activity than the glycine mutation. This suggests that the severity of precocious puberty is directly related to the degree of constitutive LH receptor activity.

Jansen's Disease: Jansen-type metaphyseal chondroplasia is a form of human dwarfism caused by abnormal bone formation and associated with parathyroid-independent hypercalcemia and hypophosphatemia. An activating mutation (histidine at 223 mutated to arginine) has been found in an affected subject which causes constitutive activation of the parathyroid receptor. This ligand-independent activation leads to abnormal endochondral bone formation in the disease and suggests that the parathyroid receptor plays an important role in normal proliferation and differentiation of growth-plate chondrocytes (Parfitt wt al. 1996).

Retinitis Pigmentosa: The four visual pigments in the human eye, rhodopsin (the rod photoreceptor) and the blue, green, and red cone opsins, are all related in structure. Like all G -protein coupled receptors, they possess seven transmembrane domains with a covalently bound retinal in transmembrane domain seven. Retinitis pigmentosa (RP) is characterized by progressive photoreceptor degeneration and blindness. Missense mutations changing lysine 296 (the site of retinal attachment) to glutamate or methionine have been identified in individuals with a form of RP thought to be due to constitutive rhodopsin activation. The loss of retinal binding is thought to relieve inhibitory constraints in the transmembrane helixes of rhodopsin and cause light-independent activation (Spiegel et al. 1995).

Hypoparathyroidism: The calcium sensing (CaS) receptor is found predominantly in parathyroid tissue but also within the kidney, brain and several other organs. Activation of the CaS receptor in parathyroid cells inhibits parathyroid hormone secretion and, in renal tubules, receptor activation leads to inhibition of calcium reabsorption. Constitutively activating mutations in this receptor are associated with a form of autosomal-dominant hypoparathyroidism (Lavlie et al. 1996). This is characterized by hypocalcemia and relatively low parathyroid hormone levels. The mutated form of the receptor exhibits increased sensitivity to extracellular calcium in comparison to the normal receptor, causing it to inhibit parathyroid hormone secretion at subnormal extracellular calcium concentrations.

Kaposi's Sarcoma: Kaposi's sarcoma is a cancer occurring commonly in human immunodeficiency virus (HIV) - infected individuals and, less commonly, in HIV - uninfected individuals. It has long been suspected that this disease was caused by an infectious agent, but only in the last few months has the agent been isolated and identified as a human gammaherpesvirus. This virus has been shown to produce a constitutively active G protein receptor which causes cancerous cell proliferation and is found in high levels within Kapsosi's sarcoma and some lymphomas (Arvanitikis et al. 1997). It is likely that the constitutive activation of this virus-related G protein-coupled receptor is directly related to tumor development.

Neuropsychiatric Diseases: An accepted pharmacological characteristic of G protein-coupled receptors is that over-production of a receptor at any site can lead to constitutive activation. The mechanism for this effect is not entirely understood but may relate to a higher than normal proportion of receptors being present in the "active" conformation in the cell membrane. This effect is very relevant to two neuropsychiatric diseases.

Schizophrenia: Although the biological basis of schizophrenia remains unknown, the ability of effective antipsychotic drugs to block the effects of dopamine in the brain suggests that overactivity of the dopamine system is implicated. In this regard, one dopamine receptor, the D4, has been found to be elevated to 600 % the normal levels in the brains of schizophrenic patients. This suggests that the D4 receptor could be constitutively active in the disease. Ligand antagonists of the dopamine D4 receptor, such as clozapine, are clinically effective anti-psychotics.

Major Depression: The ability of drugs which regulate serotonergic function to clinically relieve depression has implicated the serotonin system in the pathology of the disease. In major depressive patients, the 5-HT_{2A} receptor has been found to be overproduced in frontal cortex, a region of the brain known to be involved in controlling mood. It is likely that this over-production of the receptor is responsible for constitutive activity in this region of the brain and may contribute towards the pathophysiology of the disease. The pathophysiological relevance of this receptor is further strengthened by clinical data indicating that depressed patients responding to current antidepressants exhibit reduced 5-HT_{2A} receptor numbers after drug treatment. Those patients who did not respond to drug treatment showed no change in 5-HT_{2A} receptor numbers.

### Tyrosine Kinase Receptors:

Unlike the G protein-coupled receptors which all share the seven transmembrane motif, tyrosine kinase receptors display a broad range of structures with little similarity. As with G protein-coupled receptors, the ligand binding sites extend into the extracellular space and are joined with transmembrane portions which extend into the intracellular space. Rather than consisting of one extended protein molecule, the tyrosine kinase receptors typically consist of several independent subunits, with the ligand binding subunits distinct from the transmembrane subunits. The tyrosine kinase superfamily of receptors is divided among four subclasses of receptor, identified as classes I, II, III, and IV. Characteristic structural features of the subclasses include two cysteine-rich repeat sequences in the extracellular domain of monomeric subclass I receptors, disulfide-linked hetrotetrameric α2β2 structure with similar cysteine-rich sequences in subclass II receptors, and five or three immunoglobulin-like repeats in the extracellular domains of subclass III and IV receptors, respectively. The mode of action of thee receptors is also vastly different from the G protein-coupled receptors. In the activated state, the receptors themselves (or at least the intracellular extensions) become enzymatically active. The activated receptor may first autophosphorylate some of its own tyrosine residues which are located on the receptor section extending into the intracellular space. This auto- phosphorylation then enhances the ability of the receptor/enzyme to phosphorylate tyrosine residues in the target intracellular protein. No particular protein class, such as the G proteins, serves as the target for the phosphorylation. Despite these differences, tyrosine kinase receptors have also been found to exist in inactive and active states, and it would appear that the ligand also stabilizes tyrosine kinase receptors in the active state. Only in the active state do these receptors become enzymatic. Constitutively active forms of the tyrosine kinase receptors have also been identified. Similar to the G protein-coupled receptors, a number of diseases have been now shown to result from constitutive activation of tyrosine kinase receptors.

The existence of at least two states, one inactive, and the other active and coupled to a transduction pathway, seems to be a common organizing principle for receptors. The only variation is presented by the ion channel receptors where the active state is not coupled to a transduction pathway but represents an open pore for passage of the relevant ion. Diseases associated with constitutively active forms of the tyrosine kinase receptors and ion channel receptors are set out in Table 2 below:

**TABLE 2**

| DISEASE | RECEPTOR | RECEPTOR TYPE |
|---|---|---|
| Crouzon syndrome | Fibroblast growth factor 2 | Tyrosine Kinase |
| Achondroplasia | Fibroblast growth factor 3 | Tyrosine Kinase |
| Mast cell tumor | c-Kit | Tyrosine Kinase |
| Cancer | Met gene (HGF) | Tyrosine Kinase |
| Cancer | Erythropoietin (EPO-R) | Tyrosine Kinase |
| Cancer | Erb-2 | Tyrosine Kinase |
| Cancer | Erb-3 | Tyrosine Kinase |
| Leprechaunism/Insulin desensitization | Insulin | Tyrosine Kinase |
| Tumorigenicity | c-Mpl thrombopoetin | Tyrosine Kinase |
| Tumorigenicity | Epidermal growth factor | Tyrosine Kinase |
| Invasive properties | RON | Tyrosine Kinase |
| Chronic myelogenous leukemia (CML) | STAT 5 | Signal Transducer |
| Liddle disease (salt-sensitive hypertension) | Epithelial sodium channel | Ion Channel |

Fibroblast growth factor 2: The fibroblast growth factor receptors (FGFRs) are a family of ligand-activated, membrane-spanning tyrosine kinases. Mutations in several human FGFR genes have been identified as playing a role in certain disorders of bone growth and development. It has been reported that a cysteine 332 to tyrosine mutation found in the human FGFR results in ligand-independent constitutive activation of the receptor and leads to Crouzon syndrome (Neilson et al, 1995). Analysis of the mutant receptor protein expressed in Xenopus oocytes indicates that it forms covalent homodimers, does not bind FGF, and has increased tyrosine phosphorylation. These results indicate that the mutated receptor (FGFR-2CS) forms an intermolecular disulfide bond resulting in receptor dimerization and ligand-independent activation which plays a role in the etiology of Crouzon syndrome.

Fibroblast growth factor 3: Achondroplasia is the most common form of dwarfism which is an autosomal dominant trait resulting in a defect in the maturation of the cartilage growth plate of long bones. This has been shown to be due to a glycine to arginine substitution in the transmembrane domain of the FGF-3 receptor which results in ligand-independent constitutive activation (Webster & Donoghue, 1996). The sequence of this domain is:
VYAGILSY 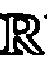 VGFFLFILVVAAVTLC
The G to R substitution is shown in outline in the transmembrane domain of the FGF-3 receptor.

c-Kit receptor: The c-kit proto-oncogene codes for a receptor which is a member of the same receptor tyrosine kinase family as platelet-derived growth factor (PDGF) and colony stimulating factor (CSF-1). The ligand for c-kit is genetically mapped to the steel (Sl) locus on mouse chromosome 10 and is variously designated as stem cell factor (SCF), mast cell growth factor, kit ligand, or steel factor. Among various types of leukemia cells tested (Kuriu et al, 1991; Ikeda et al, 1991; Furitsu et al,1993), KIT was constitutively activated in a ligand-independent manner in the HMC-1 human mast cell line (Furitsu et al, 1993). Sequencing analysis of the c-kit gene in the HMC-1 cells showed two point mutations, the V560 to G560 mutation in the juxtamembrane domain and the D816 to V816 mutation in the phosphotransferase domain. Also, a peculiar mutation has been reported in the c-Kit receptor which leads to constitutive activation in FMA3 cells (Tsujimura et al, 1996). This mutation resulted in an in-frame deletion of 21 base pairs encoding TQLPYDH at codons 573 to 579 at the juxtamembrane domain.

Met gene (HGF): The human MET proto-oncogene encodes the tyrosine kinase receptor for hepatocyte growth factor/scatter factor. MET was originally described as a human oncogene activated *in vitro* after treatment of a cell line with a chemical carcinogen (Cooper et al. 1984). Subsequently, the MET proto-oncogene was found to be amplified and/or overexpressed in a significant number of human tumors of epithelial origin (Di Renzo et al. 1991 & 1992; Prat et al. 1991; Liu et al. 1992; Natali et al. 1993). Moreover, it has been reported that transfection of a full size MET cDNA can induce transformation upon establishment of an autocrine circuit (Rong et al. 1993). The structural requirements for the constitutive activation of this receptor have been reported (Zhen et al. 1994).

Erthyropoietin receptor (EPO-R): The erythropoietin receptor (EPO-R) is a member of the cytokine receptor superfamily. A constitutively active form of the EPO-R has been isolated which has a single amino acid change in the exoplasmic domain, converting arginine 129 to cysteine (R129C). It is thought that this cysteine substitution enhances disulfide-linked dimerization which leads to ligand-independent activation . (Watowich et al. 1992). Also, aside from EPO, it has been shown that EPO-R can also be activated by the Friend spleen focus-forming virus (SFFV). Thus, the EPO-R is activated in response to multiple growth factors and oncogenes (Showers et al. 1992).

Erb-2 gene product: Overexpression of the erb-2/neu gene is frequently detected in human cancers. When overexpressed in NIH 3T3 cells, the normal erb-2 product, gp185erb-2, displays potent transforming ability as well as constitutively elevated levels of tyrosine kinase activity in the absence of exogenously added ligand (Lonardo et al. 1990). The erb-2 gene bears sequence homology to the epidermal growth factor receptor (EGFR). The expression of the erb-2 gene and EGFR have been implicated in the pathogenesis of human cancers. Their amplification or overexpression has been detected at high frequency in mammary and ovarian adenocarcinomas (Slamon et al. 1987, 1989) and in squamous cell carcinomas and glioblastomas (Xu et al. 1984; Hendler & Ozanne, 1985; Libermann et al. 1985).

ErbB-3 gene product: The predicted human erbB-3 gene product is closely related to epidermal growth factor (EGFR) and erb-2, which have been implicated as oncogenes in model systems and human neoplasia. The intrinsic catalytic function of gp180erbB-3 was shown by its ability to autophosphorylate *in vitro* (Kraus et al. 1993). These findings combined with the detection of gp180erbB-3 in 4 of 12 human mammary tumor cell lines implicate the constitutively active erbB-3 product in the pathogenesis of some human malignancies.

Insulin Receptor: The structure and function of the insulin receptor was studied in two sisters with leprechaunism. The maternal allele was deleted 3 base pairs in exon 3, causing the loss of N281 in the α-subunit (Desbios-Mouthon et al. 1996). This mutated receptor had impaired insulin binding and exhibited *in vivo* and *in vitro* constitutive activation of autophosphorylation and tyrosine kinase activity. As a result of this insulin receptor-preactivated state, the cells were desensitized to insulin stimulation of glycogen and DNA synthesis. These findings strongly suggest that N281 of the insulin receptor α-subunit plays a critical role in the inhibitory constraint exerted by the extracellular α-subunit over the intracellular kinase activity.

C-Mpl thrombopoetin receptor: c-Mpl, a receptor for thrombopoetin, belongs to the hemopoietin/cytokine receptor superfamily. It has been shown that the substitution of cysteine residues for specific amino acids in the dimer interface of c-Mpl receptor induced constitutive activation (Alexander et al. 1995). A recurring mechanism for the activation of hemopoietin receptors is the formation of functional complexes by receptor subunit oligomerization. Within the growth hormone receptor, a cluster of extracellular amino acids forms a dimer interface domain that stabilizes ligand-induced homodimers. This domain appears to be functionally conserved in the erythropoietin (EPO) receptor because substitution of cysteines for residues in the analogous region causes EPO-independent receptor activation via disulfide-linked homodimerization. The substitutions in the c-Mp1 receptor were made at the same dimer interface domain which is found in a number of homologous receptors. These results imply that the normal process of TPO-stimulated Mpl activation occurs through receptor homodimerization. Cells expressing the mutant Mpl receptors were tumorigenic in transplanted mice. Thus, like v-mpl, its viral counterpart, mutated forms of the mpl gene are oncogenic.

RON gene product: The Ron tyrosine kinase shares with the members of its superfamily (Met and Sea) the control of cell dissociation, motility, and invasion of extracellular matrices (scattering). Met is the hepatocyte growth factor receptor and shares homology to RON. RON is the human receptor for macrophage-stimulating protein (MSP). In a human gastric cancer cell line (KATO-III) an abnormal accumuladon of an uncleaved single-chain protein (delta-RON) of 165 kDa was found. This molecule is encoded by a transcript differing from the full-length RON mRNA by an in-frame deletion of 49 amino acids in the beta ("β") chain extracellular domain (Collesi et al. 1996). The mutated receptor is constitutively activated by disulfide-linked intracellular oligomerization because it contains an uneven number of cysteine residues. Oligomerization and constitutive tyrosine phosphorylation of the full-size RON was obtained by site-directed mutagenesis of a single cysteine residue in the region encoded by the cassette exon, mimicking that occurring with the naturally mutated isoform. The intracellular activation of RON is followed by acquisition of invasive properties *in vitro,* suggesting a role for the constitutively active RON receptor in the progression towards malignancy.

STAT 5: STAT-5 is a signal transducer and activator of transcription. The STAT-5 protein, which is activated transiently in normal myeloid cells by cytokines such as GM-CSF (granulocyte-macrophage colony stimulating factor), was constitutively activated in cell lines derived from patients with chronic myelogenous leukemia (Weber-Nordt et al. 1996). STAT-5 was also activated in primary mouse bone marrow cells acutely transformed by the CML-specific BCR-ABL oncogene, but not by the serine kinase oncogene v-MOS. These findings suggest a role for STAT-5 in hematopoietic transformation by BCR-ABL (Gouilleux-Gruart et al. 1996).

Epithelial sodium channel: A mutation in the epithelial sodium Channel causes a inheritable form of salt-sensitive hypertension, Liddle disease (Schild et al. 1995; Hansson et al. 1996). This mutation introduces a premature stop codon in the channel beta subunit, resulting in deletion of almost all of the C-terminus of the encoded protein. Coexpression of the mutant β-subunit with wild type alpha and gamma subunits in Xenopus laevis oocytes resulted in an approximately 3-fold increase in the macroscopic amiloride-sensitive sodium current compared with the wild type channel.

### Viral Oncogenes:

c-Kit receptor: Like many other receptor tyrosine kinases, the c-kit proto-oncogene was first identified as a retroviral oncogen (v-kit) present in the genome of the HZT feline sarcoma virus, suggesting that KIT might potentially function as an oncogenic protein, especially by structural alterations (Besmer et al. 1986).
C-Mpl thrombopoetin receptor: In the section above it is described that there is a viral counterpart to the c-Mpl TPO receptor.

### Definitions

The scientific literature which has evolved around receptors has adopted a number of terms to refer to ligands having various effects on receptors. For clarity and consistency, the following definitions will be used throughout this patent document. To the extent that these definitions conflict with other definitions for these terms, the following definitions shall control:
AGONISTS shall mean ligands which activate the intracellular response when they bind to the receptor, or enhance GTP binding to membranes.
AMINO ACID ABBREVIATIONS used herein are set out in Appendix "D".
PARTIAL AGONISTS shall mean ligands which activate the intracellular response when they bind to the receptor to a lesser degree/extent than do agonists, or enhance GTP binding to membranes to a lesser degree/extent than do agonists
ANTAGONIST shall mean ligands which competitively bind to the receptor at the same site as the agonists but which do not activate the intracellular response initiated by the active form of the receptor, and can thereby inhibit the intracellular responses by agonists or partial agonists. ANTAGONISTS do not diminish the baseline intracellular response in the absence of an agonist or partial agonist.
CANDIDATE COMPOUND shall mean a molecule (for example, and not limitation, a chemical compound) which is amenable to a screening technique. Preferably, the phrase "candidate compound" does not include compounds which were publicly known to be compounds selected from the group consisting of inverse agonist, agonist or antagonist to a receptor, as previously determined by an indirect identification process ("indirectly identified compound"); more preferably, not including an indirectly identified compound which has previously been determined to have therapeutic efficacy in at least one mammal; and, most preferably, not including an indirectly identified compound which has previously been determined to have therapeutic utility in humans.
COMPOUND EFFICACY shall mean a measurement of a the ability of a compound to inhibit or stimulate receptor functionality, as opposed to receptor binding affinity. A most preferred means of detecting compound efficacy is via measurement of [³⁵S]GTPγS binding, as further disclosed in the Example section of this patent document.
CONSTITUTIVELY ACTIVATED RECEPTOR shall mean a receptor subject to constitutive receptors activation. A constitutively activated receptor can be endogenous or non-endogenous.
CONSTITUTIVE RECEPTOR ACTIVATION shall mean stabilization of a receptor in the active state by means other than binding of the receptor with its endogenous ligand or a chemical equivalent thereof.
CONTACT or CONTACTING shall mean bringing at least two moieties together, whether in an in vitro system or an in vivo system.
DIRECTLY IDENTIFYING or DIRECTLY IDENTIFIED, in relationship to the phrase "candidate compound", shall mean the screening of a candidate compound against a constitutively activated receptor, preferably a constitutively activated orphan receptor, and most preferably against a constitutively activated G protein-coupled cell surface orphan receptor, and assessing the compound efficacy of such compound. This phrase is, under no circumstances, to be interpreted or understood to be encompassed by or to encompass the phrase "indirectly identifying" or "indirectly identified."
ENDOGENOUS shall mean a material which a mammal naturally produces.
ENDOGENOUS in reference to, for example and not limitation, the term "receptor," shall mean that which is naturally produced by a mammal (for example, and not limitation, a human) or a virus. By contrast, the term NON-ENDOGENOUS in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not limitation, in a screening approach, the endogenous or non-endogenous receptor may be in reference to an in vitro screening system. As a further example and not limitation, where the genome of a mammal has been manipulated to include a non-endogenous constitutively activated receptor, screening of a candidate compound by means of an in vivo system is viable.
INDIRECTLY IDENTIFYING or INDIRECTLY IDENTIFIED means the traditional approach to the drug discovery process involving identification of an endogenous ligand specific for an endogenous receptor, screening of candidate compounds against the receptor for determination of those which interfere and/or compete with the ligand-receptor interaction, and assessing the efficacy of the compound for affecting at least one second messenger pathway associated with the activated receptor.
INHIBIT or INHIBITING, in relationship to the term "response" shall mean that a response is decreased or prevented in the presence of a compound as opposed to in the absence of the compound.
INVERSE AGONISTS shall mean ligands which bind to either the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.
LIGAND shall mean an endogenous, naturally occurring molecule specific for an endogenous, naturally occuring receptor.
ORPHAN RECEPTOR shall mean an endogenous receptor for which the endogenous ligand specific for that receptor has not been identified or is not known.
PHARMACEUTICAL COMPOSITION shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan.
NON-ORPHAN RECEPTOR shall mean an endogenous naturally occurring molecule specific for an endogenous naturally occuring ligand wherein the binding of a ligand to a receptor activates an intracellular signalling pathway.
STIMULATE or STIMULATING, in relationship to the term "response." shall mean that a response is increased in the presence of a compound as opposed to in the absence of the compound.

### References

Alla, S.A., et al (1996). Extracellular domains of the bradykinin B2 receptor involved in ligand binding and agonist sensing defined by anti-peptide antibodies. J. Biol. Chem., 271, 1748-1755.
Alexander, W.S., et al (1995). Point mutations within the dimer interface homology domain of c-Mpl induce constitutive receptor activity and tumorigenicity. EMBO J., 14, 5569-78.
Arvanitikis, L., et al (1997). Human herpesvirus KSHV encodes a constitutively active G-protein-coupled receptor linked to cell proliferation. Nature, 385, 347-349.
Barker, E.L., et al (1994). Constitutively active 5-hydroxytryptamine 2C receptors reveal novel inverse agonist activity of receptor ligands. J. Biol. Chem., 269:16, 11687-11690.
Besmer, P., et al (1986). A new acute transforming feline retrovirus and relationship of its oncogene v-kit with the protein kinase gene family. Nature, 320, 415.
Blin, N., et al (1995). Mapping of single amino acid residues required for selective activation of Gq/11 by the m3 muscarinic acetylcholine receptor. J. Biol. Chem., 270, 17741-17748._
Burstein, E.S., et al (1995). Constitutive activation of muscarinic receptors by the G-protein Gq. FEBS Lett., 363:3, 261-3.
Casey, C., et al (1996). Constitutively active mutant 5-HT2A serotonin receptors: inverse agonist activity of classical 5HT2A antagonists. Soc.Neurosci. Abstracts # 699.10.
Chen, W., et al (1995). A colorimetric assay for measuring activation of Gs - and Gq - coupled signalling pathways. Anal. Biochem., 226:2, 349-354.
Chidiac, P., et al (1994). Inverse agonist activity of beta-adrenergic antagonists. J. Pharm. Expt. Ther., 45, 490-499.
Cheatham, B., et al (1993). Substitution of the erb-2 oncoprotein transmembrane domain activates the insulin receptor and modulates the action of insulin-receptor substrate. Proc. Natl. Acad. Sci. (USA), 90, 7336-73340.
Collesi, C., et al (1996). A splicing variant of the RON transcript induces constitutive tyrosine kinase activity and an invasive phenotype. Mol. & Cellular Biol. , 16, 5518-5526.
Cooper, C.S., et al (1984). Molecular cloning of a new transforming gene from a chemically transformed human cell line. Nature, 311, 29-33.
Desbios-mouthon, C. et al (1996). Deletion of Asn281 in the α-subunit of the human insulin receptor causes constitutive activation of the receptor and insulin desensitization. J. Clin. Endocrinol. Metab., 81, 719-727.
Di Renzo, M.F., et al (1991). Expression of the Met/HGF receptor in normal and neoplastic human. Oncogene, 6:11, 1997-2003.
Di Renzo, M.F., et al (1992). Overexpression of the c-Met/HGF receptor gene in human thyroid carcinomas. Oncogene, 7, 2549-2553.
Duprez, L., et al (1994). Germline mutations in the thyrotropin receptor gene cause non-autoimmune autosomal dominant hyperthyroidism. Nature Genetics, 7, 396-401.
Eggerick, D., et al (1995). Molecular cloning of an orphan G-protein-coupled receptor that constitutively activates adenylate cyclase. Biochem. J., 389, 837-843.
Furitsu, T., et al (1993). Identification of mutations in the coding sequence of the proto-oncogene c-kit in a human mast cell leukemia cell line causing ligand-independent activation of c-kit product. J. Clin. Invest., 92, 1736.
Gouilleux-Gruart, V., (1996). STAT-related transcription factors are constitutively activated in peripheral blood cells from acute leukemia patients. Blood, 87:5, 1692-7.
Hansson, J.H., et al (1995). Hypertension caused by a truncated epithelial sodium channel gamma subunit: genetic heterogeneity of Liddle syndrome. Nat. Genet., 11:1, 76-82.
Hasegawa, H., et al (1996). Two isoforms of the prostaglandin E receptor EP3 subtype different in agonist-independent constitutive activity. J. Biol. Chem., 271:4, 1857-1860.
Hendler, A.M. & Ozanne, B.W. (1984). Human squamous cell lung cancers express increased epidermal growth factor receptors. J. Clin. Invest., 74, 647-651.
Herrick-Davis, K., et al (1996). Constitutively active 5HT2c serotonin receptor created by site directed mutagenesis. Soc. Neuroscience abstract #699.18.
Hogger, P. et al (1995). Activating and inactivating mutations in the N- and C-terminal I3 loop junctions of muscarinic acetylcholine Hm1 receptors. J. Biol. Chem., 270, 7405-7410.
Ikeda, H., et al (1991). Expression and functional role of the proto-oncogen c-kit in acute myeloblastic leukemia cells. Blood, 78, 2962.
Kjelsberg, M.A., et al (1992). Constitutive activation of the alpha 1B-adrenergic receptor by all amino acid substitutions at a single site. J. Biol. Chem., 267, 1430-1433.
Kosugi, S., et al (1995). Characterization of heterogenous mutations causing constitutive activation of the luteinizing hormone receptor in familial male precocious puberty. Human Molecular Genetics, 4:2, 183-188.
Kraus, M.H., et al (1993). Demonstration of ligand-independent signalling by the erbB-3 tyrosine kinase and its constitutive activation in human breast tumor cells. Proc. Natl. Acad. Sci. (USA), 90, 2900-4.
Kuriu, A., et al (1991). Proliferation of human myeloid leukemia cell line associated with the tyrosine phosphorylation and activation of the proto-oncogene c-kit product. Blood, 78, 2834.
Latronico, A.C., et al (1995). A novel mutation of the luteinizing hormone receptor gene causing male gonadotropin-independent precocious puberty. J. Clin. Endocrinol. Metab., 80, 2490-2494.
Laue, L., et al (1995). Genetic heterogeneity of constitutively activating mutations of the human luteinizing hormone receptor in familial male-limited precocious puberty. Proc. Natl. Acad. Sci. (USA), 92, 1906-1910.
Lavlie, R., et al (1996). The Ca(2+)-sensing receptor gene (PCAR1) mutation T151M in isolated autosomal dominant hypoparathyroidism. Hum. Genet., 98:2, 129-33.
Lefkowitz, R., et al (1993). Constitutive activity of receptors coupled to guanine nucleotide regulatory proteins. Trends Pharmacol. Sci., 14, 300-307.
Libermann, T.A., et al (1985). Amplification, enhanced expression and possible rearrangement of EGF receptor gene in primary human brain tumors of glial origin. Nature, 313, 144-147.
Liu , C., et al (1992). Overexpression of c-met proto-oncogene but not epidermal growth factor receptor or c-erbB-2 in primary human colorectal carcinomas. Oncogene, 7:1, 181-185.
Liu, J., et al (1996). Molecular mechanisms involved in muscarinic acetylcholine receptor-mediated G protein activation studied by insertion mutagenesis. The J. Biol. Chem., 271:11, 6172-6178.
Lonardo, F., et al (1990). The normal erb-2 product is an atypical receptor-like tyrosine kinase with constitutive activity in the absence of ligand. The new Biologist, 2:11, 992-1003.
Maenhault, C., et al (1990). RCD8 codes for an adenosine A2 receptor with physiological constitutive activity. Biochem. Biophys: Res. Com., 173:3, 1169-1178.
Myles, G.M., et al (1994). Tyrosine 569 in the c-fms juxtamembrane domain is essential for kinase activity and macrophage colony-stimulating factor-dependent internalization. Mol. Cell. Biol., 14, 4843.
Natali, P.G., et al (1993). Expression of the c-Met/HGF receptor in human melanocytic neoplasms: demonstration of the relationship to malignant melanoma tumor progression. Br. J. Cancer, 68:4, 746-750.
Neilson, K.M., et al (1995). Constitutive activation of fibroblast growth factor receptor-2 by a point mutation associated with Crouzon syndrome. J. Biol. Chem., 270:44, 26037-26040.
O'Dowd, B.F., et al (1988). Site-directed mutagenesis of the cytoplasmic domains of the human BETA2-adrenergic receptor. J. Biol. Chem., 263, 15985-15992.
Parent, J., et al (1996). Mutations of two adjacent amino acids generate inactive and constitutively active forms of the human platelet-activating factor receptor. J. Biol. Chem., 271:14, 7949-7955.
Parfitt, A.M., et al (1996). Hypercalcemia due to constitutive activity of the parathyroid hormone (PTH)/PTH-related peptide receptor: comparison with primary hyperparathyroidism. J. Clin. Endocr. Metab., 81, 3584-3588.
Parma, J., et al (1993). Somatic mutations in the thyrotropin receptor gene cause hyperfunctioning thyroid adenomas. Nature, 365, 649-651.
Pei, G., et al (1994). A constitutive active mutant BETA2-adrenergic receptor is constitutively desensitized and phosphorylated. Proc. Natl. Acad. Sci. (USA), 91, 2699-2702.
Prat, M.P., et al (1991). The receptor encoded by the human C-MET oncogene is expressed in hepatocytes, epithelial cells and solid tumors. Int. J. Cancer, 49, 323-328.
Prezeau, L., et al (1996). Changes in the carboxy-terminal domain of metabotropic glutamate receptor 1 by alternate splicing generate receptors with differing agonist-independent activity. Mol., Pharmacol., 49, 422-429.
Ren, Q., et al (1993). Constitutive active mutants of the ALPHA2-adrenergic receptor. J. Biol. Chem., 268, 16483-16487.
Rong, S., el al (1993). Met expression and sarcoma tumorigenicity. Cancer Res., 53:22, 5355-60.
Scheer, A., et al (1997). The activation process of the a1B-adrenergic receptor: potential role of protonation and hydrophobicity of a highly conserved aspartate. Proc. Natl. Acad. Sci. (USA)., 94, 808-813.
Schwinin, D.A., et al (1995). Cloning and pharmacological characterization of human Alpha-1 adrenergic receptors: sequence corrections and direct comparison with other species homologues. The J. Pharmacol., 272, 134-142.
Schild, L., et al (1995). A mutation in the epithelial sodium channel causing Liddle disease increases channel activity in the Xenopus laevis oocyte expression system. Proc. Natl. Acad. Sci. (USA), 92, 5699-703.
Sharif, M., et al (1994). Malignant transformation by G protein-coupled hormone receptors. Molecular & Cellular Endocrinology, 100, 115-119.
Showers, M.O., et al (1992). Activation of the erythropoietin, receptor by the Friend spleen focus-forming virus gp55 glycoprotein induces constitutive protein tyrosine phosphorylation. Blood, 80, 3070-8.
Skinner, R.H., et al (1994). Direct measurement of the binding of Ras to neurofibromin using scintillation proximity assay. Anal. Biochem., 223, 259-265.
Slamon, D.J., et al (1987). Human breast cancer: correlation of relapse and survival with amplification of the HER-2 neu oncogene. Science, 235, 177-182.
Slamon, D.J., et al (1989). Studies of the HER-2/neu proto-oncogene in human breast and ovarian cancer. Science, 244, 707-712.
Solomon, Y., et al (1974). A highly sensitive adenylate cyclase assay. Anal. Biochem., 58, 541-548.
Spiegel, A.M., et al (1995). Defects in G protein-coupled signal transduction in human disease. Ann. Rev. Physiol., 58, 143-170.
Ter Lack, A., et al (1995). Modelling and mutation studies on the histamine H1-receptor agonist binding site reveal different binding modes for H1-agonists: Asp116 (TM3) has a constitutive role in receptor stimulation. J. Computer-aided molecular design, 9, 319-330.
Tsujimura, T., et al (1996). Constitutive activation of c-kit in FMA3 murine mastocytoma cells caused by a deletion of seven amino acids at the juxtamembrane domain. Blood, 87, 273-283.
Watowich, S.S., et al (1992). Homodimerization and constitutive activation of the erythropoietin receptor. Proc. Natl. Acad. Sci. (USA), 89, 2140-4.
Weber-Nordt, R.M., et al (1996).Constitutive activation of STAT proteins in primary lymphoid and myeloid leukemia cells and in Epstein-Barr virus (EBV)-related lymphoma cell lines. Blood, 88:3, 809-16.
Webster, K. & Donoghue, J. (1996). Constitutive activation of fibroblast growth factor receptor 3 by the transmembrane point mutation found in achondroplasia. The EMBO J., 15, 520-527.
Xu, Y.H., et al (1984). Characterization of epidermal growth factor receptor gene expression in malignant and normal human cell lines. Proc. Natl .Acad. Sci. (USA)., 81, 7308-7312. Yamada, K., et al (1992). Substitution of the insulin receptor transmembrane domain with the c-neu/erb2 transmembrane domain constitutively activates the insulin receptor tyrosine kinase in vitro. J. Biol. Chem., 267, 12452-12461.
Zhen, Z., et al (1994). Structural and functional domains critical for constitutive activation of the HGF-receptor (Met). Oncogene, 9, 1691-1697.

### BRIEF SUMMARY OF THE INVENTION

Disclosed herein are methods for directly identifying candidate compounds as inverse agonists, partial agonists or agonists (most preferably inverse agonists) to orphan G protein coupled receptors comprising a mutation in its amino acid sequence so as to render it a non-endogenous constitutively activated orphan G protein-coupled receptor. Use of the disclosed method allows for direct identification of candidate compounds as inverse agonsits, partial agonists or agonists (most preferably inverse agonists) to the receptor without prior knowledge of the endogenous ligands specific for such receptors.

Further understanding and appreciation of the invention disclosed herein will become apparent from the detailed description which follows.

### BRIEF DESCRIPTION OF THE DRAWINGS

Figure 2 shows a generalized structure of a G protein-coupled receptor with the numbers assigned to the transmembrane helixes, the intracellular loops, and the extracellular loops.
Figure 1 schematically shows the two states, active and inactive, for a typical G protein-coupled receptor and the linkage of the active state to the second messenger transduction pathway.
Figure 3 shows some of the known constitutively activated receptors along with 1) any associated disease; 2) the nature of the constitutive activation (sequence modification, antibody, overexpression); 3) the location of the activating modification; 4) the nature of the activating modification; and 5) reference citations.
Figure 4 is a diagram showing the increased affinity of constitutively active human 5-HT_{2C} receptors for serotonin by its enhanced ability to compete with ³H-LSD binding to the native and constitutively active serotonin 5-HT_{2C} receptors.
Figure 5 is a diagram showing that the (non-endogenous) human 5-HT_{2C} (S310K) mutant receptor shows enhanced intracellular accumulation of inositol tris phosphate in COS cells relative to the endogenous 5-HT_{2C} receptor and cells transfected with control alone. The endogenous receptor also shows a high degree of natural constitutive activity.
Figure 6 is a diagram showing the enhanced binding of [³⁵S]GTPγS to membranes prepared from 293T cells transfected with the endogenous and non-endogenous (S310K) 5-HT_{2C} receptors compared to those transfected with control vector alone at 75 µg/well membrane protein. The radiolabeled concentration of [³⁵S]GTP-γS was held constant at 1.2 nM and GDP concentration was held constant at 1 µM. The assay was performed on 96-well format in Wallac scintistrips.
Figure 7 is a diagram showing enhanced [³⁵S]GTPγS binding to membranes prepared from COS cells expressing the endogenous 5-HT_{2C} receptor in response to serotonin and inhibition by mianserin using wheatgerm agglutinin scintillation proximity beads. The concentration of [³⁵S]GTPγS was held constant at 0.3 nM, and the concentraion of GDP was held at 1 µM. The concentration of the membrane protein was 12.5 µg.
Figure 8 is a diagram showing serotonin stimulation of [³⁵S]GTPγS binding to membranes expresing the non-endogenous (S310K) constitutively active 5-HT_{2C} receptor in 293T cells and inhibition by 30 µM mianserin on Wallac scintistrips.
Figure 9 is a diagram showing the effects of protein conentration on [³⁵S]GTPγS binding in membranes prepared from 293T cells transfected with the endogenous and non-endogenous (S310K) 5-HT_{2C} receptors compared to cells transfected with the control vector alone in the absence (A) and presence (B) of 10 µM serotonin. The radiolableled concentration of [³⁵S]GTP-γS was held constant at 0.3 nM, and the GDP concentraion was held constant at 1 µM. The assay was performed on 96-well format in Wallac scintistrips.
Figure 10 is a diagram showing enhanced binding of [³⁵S]GTP-γS to membranes prepared from 293T cells transfected with the orphan receptor GPR3 compared to those transfected with control vector alone at 75 µg/well membrane protein. The radiolabeled concentration of [³⁵S]GTP-γS was held constant at 1.2 nM and the GDP concentration was held constant at 1 µM. The assay was performed on 96-well format in Wallac scintistrips.
Figure 11 is a diagram showing the effects of [³⁵S]GTPγS concentration on binding in membranes prepared from 293T cells transfected with the endogenous 5-HT_{2C} receptor, the non-endogenous constitutively active 5-HT_{2C} receptor, the orphan receptor GPR3, and the control vector alone at 37.5 (A) and 75 (B) µg/well of membrane protein. The GDP concentration was held constant at 1 µM. The assays was performed on 96-well format in Wallac scintistrips. Figure 12 shows the amino acid alignment of orphan receptors GPR3, GPR6, and GPR12.
Figure 13 is a diagram showing that the orphan receptors GPR3, GPR6, and GPR 12 are confirmed to be constitutively active by their enhanced ability to induce expression of β-galactosidase from a CRE driven reporter system in VIP cells following the procedure of Konig (see Example 3).
Figure 14 shows the relative distribution of the expression of the GPR3 (A), GPR6 (B), and GPR12 (C) orphan receptors across several normal human tissues as determined by RT-PCR. Abbreviations: Ocx = occipital cortex; Hypoth = hypothalamus; Tex = temporal cortex; Fcx = frontal cortex.
Figure 15 shows GPR3 receptor expression in normal and epileptic human brain tissue as examined by RT-PCR. The control sample is on the left and the epileptic sample is on the right.
Figure 16 shows the plate profile after screening candidate compounds at the orphan G protein-coupled receptor, GPR 3. Compounds in wells P44 and P45 are inverse agonists.
Figure 17 shows the plate profile after screening candidate compounds at the orphan G protein-coupled receptor, GPR 6. Compounds in wells P3, P13, P15, P18, P20, P32, P33, P36, P55, P66, P67, P69, and P70 are inverse agonists.

### DESCRIPTION OF THE PREFERRED EMBODIMENTS

In modem molecular biology, important advances have frequently occurred when an individual stepped outside of the traditionally accepted way of viewing things and examined a situation afresh from a new perspective. Typically, when such circumstances have occurred, all the information with which to deduce the insight has been present, but has been unappreciated except by that particular person. Two prominent examples of this phenomenon are the discoveries of the reverse information flow from RNA to DNA and the technique of DNA amplification known as PCR. Nothing in the original discovery of the transfer of genetic information from DNA to RNA ever proscribed the transfer of genetic information from RNA to DNA. Yet, so elegant and successful were the results of following the information flow from DNA that the DNA to RNA pattern became an entrenched model, and it was not until someone stepped outside of the traditional model and asked what was needed for information to flow in the opposite direction that it was recognized that RNA carrying viruses could integrate genetic information into the DNA of infected cells. This invention disclosed in this patent document arises from just such a unique shift in perspective.

With the single exception mentioned earlier, the traditional study of receptors has always proceeded from the a priori assumption (historically based) that the endogenous ligand must first be identified before discovery could proceed to find antagonists and other molecules which could affect the receptor. Even in cases where an antagonist might have been known first, the search immediately extended to looking for the native agonist. This mode of thinking has persisted in receptor research even after the discovery of constitutively activated receptors. What has not been heretofore recognized is that it is the active state of the receptor which is most useful for discovering agonists, partial agonists, and inverse agonists of the receptor. For those diseases which result from an overly active receptor, which includes all those summarized earlier, what is desired in a therapeutic drug is a compound which acts to diminish the active state of a receptor, not necessarily a drug which is an antagonist to the normal ligand. This is because a compound (drug) which reduces the activity of the active receptor state need not bind at the same site as the endogenous ligand. Thus, as taught by a method of this invention, any search for therapeutic compounds should start by screening compounds against the ligand-independent active state. The search, then, is for an inverse agonist to the active state receptor. Constitutively active receptors are, therefore, most preferred. An additional feature of an approach of this invention is that inverse agonists to the endogenous receptor can be directly identified .

Screening candidate compounds against the endogenous or non-endogenous constitutively activated receptor allows for the direct identification of candidate compounds which act at orphan or known cell surface receptors without requiring any prior knowledge or use of the receptor ligand. This feature of a method of this invention is particularly vital for the orphan receptors which are being identified by the Human Genome Project. As such, this method is useful for accelerating drug discovery at a broad range of orphan receptors. It has been estimated that 2 % of the human genome encodes G protein-coupled receptors (about 2000 distinct proteins), the very large majority of which are orphan receptors. A method of this invention provides a previously undisclosed means to identify new drugs acting at this large family of receptors, as well as at other types of receptors disclosed herein.

In addition to the ability of this method to capitalize on existing and emerging genetic information on orphan receptors, drugs developed using the method disclosed herein at known receptors possess distinct therapeutic advantages over those developed using traditional screens. Ligand-based screens only identify compounds which act to "antagonize" the action of the ligand on the receptor and thereby prevent ligand-induced receptor activation. Such compounds, or antagonists, do not block ligand-independent receptor activation and the subsequent cellular response. On the other hand, drugs developed using the method of this invention, most preferably block both ligand-dependent and ligand-independent receptor activation. This property of drugs identified with the methodology of this invention is extremely relevant to the growing number of diseases which have been linked to constitutively active G protein coupled receptors (Figure 3) since traditional antagonists generally will not block such activity.

The fundamental insight underlying the present invention is the recognition that it is the constitutively activated form of receptors which can be used to directly identify lead compounds which affect receptor activity. For the case of a disease caused by a constitutively activated receptor, a small molecule inverse agonist to the constitutively activated receptor is, in itself, a potential therapeutic drug. For the case of any receptor, but particularly for orphan receptors, the likelihood of discovery of additional agonists and the endogenous ligand is increased significantly using the constitutively activated receptor since the receptor displays an enhanced response to such agonists. Inverse agonists to the orphan receptors can also be directly identified. The search for the biological function of orphan receptors will be greatly simplified when both agonists and inverse agonists to orphan receptors are available. Essentially, the method of this invention provides a means for discovering modulators of receptor function without the need for any knowledge of the endogenous ligand. This ability is without counterpart in the prior art.

Utilization of the invention disclosed herein relies upon obtaining or producing constitutively activated receptors. The great advances in the science of genetic manipulation and engineering which have occurred over the past few years now make available on a routine basis techniques of genetic identification, construction, and expression which only a short time ago required a practitioner highly skilled in the art. Routine methods are now well known to practitioners of the genetic arts for altering Genetic sequences of DNA which will direct cells to produce proteins having desired amino acid sequences. The methods for introducing the altered DNA into cells and inducing the cells to express the altered protein specified by the altered DNA are also well established. An established reference work describing these techniques is that of Sambrook Fritsch and Maniatis, 1984, Molecular Cloning, A Laboratory Manual*.*

As indicated above, the sequences of constitutively activated receptors associated with some diseases are known and genetic material encoding many of these sequences is available. In those cases in which the genetic material is not available, a constitutively activated receptor identical to the disease form may be produced by introducing the same constitutive activating mutation as is found in the wild type constitutively activated receptor. However, many more known receptors have not yet been associated with a disease state, and the orphan receptors, by definition, have no known function. For these receptors, it is first necessary to induce constitutive activation. In Section "A" set out below, a number of approaches to constitutively activating receptors are set forth. These approaches are based upon a detailed analysis and synthesis of the naturally occurring activation patterns which have been observed. No single approach is a most preferred approach for any given receptor, in that, based upon the disclosure of this invention, these approaches provide the skilled artisan the opportunity to select, without undue experimentation and predicated upon the needs of the artisan, one or more approaches which will lead to a constitutively activated form of a given receptor.

In Section "B" set out below, transfecting the constitutively activated receptor into an appropriate *in vitro* expression system (mammalian or non-mammalian) is disclosed. As mentioned above, standard transfection techniques are well known in the art with which to perform this step. (Chidiac et al. 1994). Successful transfection of a constitutively active receptor should always be confirmed by appropriate tests and one such test is set forth.

In Section "C" set out below, confirmation of the constitutive activity of the transfected receptor in the expression system is disclosed. A variety of second messenger screening assays can be employed to detect the receptor-mediated cellular response. The assay chosen primarily depends upon the type of receptor and the secondary pathway it activates. For example, for some G protein-coupled receptors an adenyl cyclase activated system would provide the appropriate assay. For other G protein-coupled receptors, a phospholipase C linked assay would be appropriate. Appropriate assays for tyrosine kinase and other receptors are available and known to those skilled in the art. Preferred assays are summarized below. The assays of constitutively activated receptor activity not only demonstrate the functioning of the receptor activity, but they also provide a means to directly determine when the level of that activity has been decreased or increased. Thus, compounds which are inverse agonists would be expected to lower the observed basal level of activity while compounds which are agonists would be expected to increase the activity level above baseline.

Constitutively activated receptors in an *in vitro* expression system can, therefore, form the basis of screening assays by which candidate compounds may be directly identified. Using expressed constitutively activated receptor systems as reagents, candidate compounds can applied to the reagent system and the assay systems used to directly identify compounds which act upon the constitutively activated receptor. The method of this invention solves a major problem of finding pharmacologically effective compounds for regulation of receptor activity even in the absence of any prior knowledge about the endogenous ligand to the receptor.

Once it has been appreciated that: 1) inverse agonists to orphan receptors can be identified by the methodologies of this invention; and 2) that such inverse agonists are ideal candidates as lead compounds in drug discovery programs for treating diseases related to the receptors, a search, impossible in the prior art, for treatments to diseases becomes enabled by this knowledge. For example, scanning both diseased and normal tissue samples for the presence of a receptor now becomes more than an academic exercise or one which might be pursued along the path of identifying an endogenous ligand. Since, by definition, the endogenous ligand for an orphan receptor is not known, tissue scans can be conducted across a broad range of healthy and diseased tissues. Such tissue scans provide a preferred first step in associating a specific receptor, for which modulating compounds are now known, with a disease. The DNA sequence of a receptor may be used to make a probe for RT-PCR identification of the expression of the receptor in the tissue samples. The presence of the receptor in a diseased tissue, or the presence of the receptor at elevated concentrations in diseased tissue compared to a normal tissue strongly can be preferably utilized to identify a correlation with that disease. Receptors can equally well be localized to regions of organs by this technique. Based on the known functions of the specific tissues to which the receptor is localized, the putative functional role of the receptor can be deduced.

A further example of a type of fruitful search which may now be undertaken with knowledge of the methodologies of the present invention is the identification and association with diseases of classes of receptors identified by their DNA homology with specific receptors. The mutation producing activation in a first receptor may also produce activation in homologous receptors. Thus, by using, preferrably, known computerized databases, the sequence of the known receptor can be effectively utilized to obtain families of related receptors. Normal and disease tissue samples can, in turn, be screened for an understanding of the location of these receptor homologs. Since, inverse agonists can also be identified with the methodologies of the present invention for these homologous receptors, their identification and distribution in tissue types provides yet another approach to developing new drugs. While this approach can be utilized with receptors both for which an endogenous ligand is known and for orphan receptors, the major advance in the state of the art enabled by the methodologies of the present invention is the ability to directly identify candidate compounds for diseases associated with orphan receptors. This ability creates a whole new paradigm for drug discovery.

The steps summarized above will be highlighted below in sufficient detail to allow one skilled in the relevant art to practice the method of the invention. While, for purposes of illustration, the following discussion centers around two of the major classes of receptors, G protein-coupled receptors and tyrosine kinase receptors, it should be understood that the approach and method of this invention can equally well be used with other classes of receptors.

The following is intended to elucidate preferred approaches, and is neither intended, nor is to be construed, to be understood as a limitation on the invention disclosed herein. The order of the following steps is for presentational efficiency and is not a limitation on the manner by which the steps are to be performed, e.g., one of ordinary skill in the art may desire, following review of this patent disclosure, to adjust the steps in a different order as a matter of convenience.

### I. PRODUCTION OF NON-ENDOGENOUS CONSTITUTIVELY ACTIVATED FORM OF TARGET RECEPTOR

### A. Molecular Alterations of Target Receptors

### 1. Non-endogenous, constitutively activated G Protein-Coupled Receptors

Constitutively active receptors can be produced by mutating single amino acids in target receptors or by substituting selected stretches of amino acids, referred to as "mutational cassettes", into the receptor. In this regard, there are certain identified regions (and single amino acid positions) within the G protein-coupled receptor (GPCR), which as the data set forth herein indicated, produce a constitutively active form of the receptor.

### a. Mutational Cassettes

### (1) Cassettes of The Second Extracellular (E2) and Third Intracellular (3) Loops

A number of G protein-coupled receptors have been reported to become constitutively active after amino acid mutations have occurred or have been engineered into the receptor sequence. Important loci for these mutations are the second extracellular and third intracellular loops. Disclosed below are some mutational cassettes, which consist of a short segments of amino acids based upon observed mutations, which will be used to engineer constitutive activity into orphan G protein-coupled receptors. Each cassette is discussed in conjunction with the receptor/receptors from which it was identified.

### (a) β-adrenergic Receptor

Receptors found to belong to the adrenoreceptor class of G protein-coupled receptors can be constitutively activated by a short mutational cassette covering the C-terminal portion of the third cytoplasmic loop (amino acids 264-272, see below). Substitution of this cassette was able to constitutively activate Gs- and Gi -coupled adenylate cyclase linked and Gq-coupled phosphoinositol linked receptors (reviewed in Lefkowitz et al. 1993). This mutational cassette will be used to activate a range of orphan G protein-coupled receptors.
Activating Mutational Cassette: (264-272) FCSREKKAA
Activating mutational cassette for whole third intracellular loop:
RVFQEAKRQLQKIDKSEGRFHVQNLSQVEQDGRTGHGLRRSSKFC 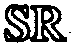 SRE 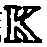 KA 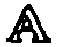 KT
   In addition to the shorter cassette, the whole third intracellular loop containing the shorter cassette of this receptor (shown above) corresponding to amino acids 264-272 will also be used in the practice of the invention. Thus, in one embodiment of the invention, the cDNA sequence corresponding to these cassettes, either the shorter one or the longer one, will be engineered into the third intracellular loop of G protein-coupled receptors.

### (b) α1B Adrenergic Receptor

Mutations in the intracellular region of the α1B adrenergic receptor have been shown to constitutively activate the receptor, resulting in G protein coupling in the absence of agonist.

Mutational cassette: SREKKA 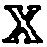 KTL where 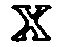 is an amino acid other than A. Most preferably, 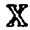 is E in that the maximum constitutive activation is obtained when E is substituted for 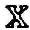 .

(See Kjelsberg et al. 1992)

The mutational cassette for the whole third intracellular loop is:
RVYIVAKRTTKNLEAGVMKEMSNSKELTLRIHSKNFHEDTLSSTKAKGHNPRS SIAVKLFKFSREKKA 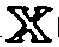 KTL where 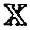 is an amino acid other than A. Most preferably, 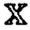 is E in that the maximum constitutive activation is obtained when E is substituted for 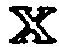 .

Thus, in another embodiment of this invention, the cDNA sequence corresponding to this cassette (with variations at 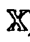 ) will be engineered into the third intracellular loop of G protein-coupled receptors. (For the sequence of the third intracellular loop, see Schwinin et al. 1995.)

### (c) ACCA (adenylate cyclase constitutive activator)

Recently, Eggerick et al. 1995 reported the molecular cloning of an orphan G protein-coupled receptor that constitutively activates adenylate cyclase which was called ACCA or adenyl cyclase constitutive activator. Abundant transcripts were found in the brain, whereas lower amounts were detected in the testis, ovary and eye. The method of this invention is applicable to discovering inverse agonists acting at this receptor. In addition, mutational cassettes corresponding to either the second extracellular or third intracellular loops of this receptor have utility for constitutively activating other orphan receptors in the practice of this invention. Two such cassettes are set out below:
Second extracellular loop mutational cassette: CLDGLTTCGVVYPLSKNHLVVL
Whole third intracellular loop sequence: CRIVCRHAQQIALQRHLLPASHYVATRKG
cDNAs corresponding to these amino acid sequences will be used, in yet another embodiment of this invention, to replace the second extracellular and third intracellular loops of diverse orphan receptors to constitutively activate them. Transmembrane mutational cassettes ,corresponding to the transmembrane segments of this receptor will also be useful in the practice of this invention.

### (d) Serotonin Receptors

In the serotonin 5-HT_{2A} receptor, mutation of cysteine 322 to K, E or R results in constitutive activation. In addition, mutation of S312 to F or K in the 5-HT_{2C} receptor leads to constitutive activation.

### (e) The Thyrotropin Receptor

Germline mutations in the third intracellular loop of the thyrotropin receptor gene cause toxic adenomas by constitutive activation of the receptor (Duprez et al. 1994). A cassette based on this mutation is set out below.
YITVRNPQYNPGDK 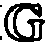 TKI 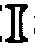 KR D619G; A623I
Also, Parma et al reported another activating mutation in the third loop which was at the same position but mutated D to C (Parma et al. 1993). A cassette based on this mutation is set out below:
YITVRNPQYNPGDK 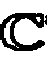 TKI 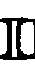 KR D619C; A623I

Thus, in another embodiment of this invention, these cassettes will be used to activate other G protein-coupled receptors.

### (f) The RDC8 Adenosine A2 receptor

The RDC8 receptor codes for a constitutively active form of the adenosine A2 receptor (Maenhaut et al. 1990). Mutational cassettes corresponding to the second extracellular and third intracellular loops of this receptor will be introduced into other receptors to provide constitutively activated receptors for the practice of this invention.

### (g) The Parathyroid Hormone (PTH) receptor

A constitutively active form of the PTH receptor has been shown to cause Jansen's disease, a rare form of short-limbed dwarfism (Parfitt et al. 1996). The constitutively active form has a mutation of T410P. In addition, another mutation, H223R, has been shown to result in the disease. Thus, mutational cassettes containing these mutations will be used to activate other receptors in a further embodiment of this invention.

### (h) Muscarinic receptors

It has been reported that a four amino acid motif (VTIL) on the m2 muscarinic receptor (corresponding to V385, T386, I389, and L390) is essential for G_{i/o} coupling specificity and G_{i/o} activation. To test the hypothesis that structural changes in this region might involve a relative movement of transmembrane VI toward the cytoplasm, a series of mutant m2 muscarinic receptors were made by Liu et al in which one to four extra alanine residues were inserted into transmembrane VI immediately after L390 (Liu et al. 1996). Consistent with the hypothesis, all mutant m2 receptors containing extra alanine mutations from the C-terminal to the L390 residue were constitutively active.

The m2 carboxy terminal region of 3rd intracellular loop is as follows:
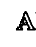 LITRTVKK Insertion of 1,2,3 or 4 alanine residues after A390 results in constitutive activation.
Thus, in a further embodiment of this invention, by sequence alignment with other G-protein coupled receptors, alanine insertion at this site will be used to constitutively activate G protein-coupled receptors.

### (2) Transmembrane cassettes

### (a) The Thyrotropin Stimulating Hormone (TSH) Receptor

Germline mutations in the thyrotropin (TSH) receptor gene cause non-autoimmune autosomal dominant hyperthyroidism (Duprez et al. 1994). It has been reported that two different mutations in the TSH receptor gene of affected members of two large pedigrees with non-autoimmune autosomal dominant hyperthyroidism (toxic thyroid hyperplasia) involve residues in the third (V509A) and seventh (C672Y) transmembrane segments. Thus, in a further embodiment of this invention, mutational cassettes corresponding to the third and seventh transmembrane domains containing these mutations of the thyrotropin receptor set out below will be used for replacing the third and seventh transmembrane regions of other receptors to constitutively activate them.
Third transmembrane mutational cassette: NTAGFFTVFASELS 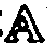 YTLTVTTLE V509A
Seventh transmembrane mutational cassette: ILLVLFYPLNS 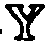 ANPFLYAIFTKA C672Y

### (b) The Histamine H1-receptor

It has been reported that in the histamine H1 receptor the D116 in transmembrane 3 is important for constitutive activation (Ter lack et al. 1995). D116, which is highly conserved in aminergic G protein-coupled receptors is generally seen as the main anchoring point for agonist and antagonist binding. In yet another embodiment of this invention, a point mutation at this site will be used to induce constitutive activation in other receptors.

### (c) Luteinizing hormone receptor

A number of natural mutations in the luteinizing hormone (LH) receptor have been shown to cause familial male precocious puberty (Kosugi et al. 1995). One mutation involves a substitution of D578 for a G in transmembrane helix 6. Thus, the G substituted transmembrane helix 6 of the LH receptor is believed to be a useful substitution for transmembrane 6 of other receptors to constitutively activate them. Other mutations corresponding to M571 for I at the cytoplasmic end of transmembrane helix 6, and T577 for I, also result in constitutive activation. Both of these mutational cassettes will be used in further embodiments of this invention to constitutively activate G protein-coupled receptors.

### (d) Viral oncogenes - Human herpesvirus GPCR

The human herpesvirus 8 has been shown to code for a constitutively active G protein-coupled receptor which is thought to be responsible for the progression of Kaposi's sarcoma, lymphomas and other malignancies (Arvanitikis et al. 1997). Mutational cassettes from' this receptor corresponding to the second extracellular, second intracellular or third intracellular loops will, in a further embodiment of this invention, provide a useful tool for activating G protein-coupled receptors. The transmembrane segments of this receptor will also be useful.

### b. Truncation of Carboxy-Terminal Tail

A number of naturally occurring alternatively spliced forms of the prostaglandin E receptor EP3 subtype are made constitutively active by truncation of the C-terminal tails (Hasegawa et al. 1996). These truncated receptors constitutively activate Gi in the absence of ligand.

In addition, the metabotropic glutamate receptors (mGluRs) share no sequence homology and show different structural features compared with most other G protein-coupled receptors. In particular, some isoforms of the phospholipase C (PLC)-coupled mGluRs (mGluR1a, mGluR5a, and mGluR5b) have surprisingly long carboxy-terminal intracellular domains, whereas splice variants mGluR1b and mGluR1c have a much shorter carboxy terminus. Expression of the short forms of mGluR1b and mGluR1c did not modify basal inositol phosphate production. (see Prezeau et al. 1996). In contrast, expression of similar levels of mGluRla resulted in a 2-fold increase in the basal inositol phosphate formation. These data suggest that for the case of metabotropic glutamate receptors, long carboxy-terminal domains favor coupling to G proteins. Thus, in another embodiment of this invention, G-protein coupled receptors will be constitutively activated by appropriate modification (truncation or extension) of the carboxy-terminal domain.

### c. Point Mutations

In many of the mutational cassettes listed above, a change in a single amino acid has resulted in constitutive activation. Thus, point mutations in the second extracellular, second intracellular, and third intracellular loops and in the transmembrane domains of seven transmembrane G protein-coupled receptors will also be used to constitutively activate these receptors.

A quantitative approach has been used to investigate the role of D142 which belongs to the highly conserved 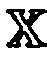 RY (where 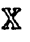 is normally either E or D) sequence in the activation process of the α1B-adrenergic receptor. The activation process depended on the protonation state of D142. D142 of the α1B-adrenergic receptor was mutated into all 19 possible amino acids and receptor mutants were tested for their ability to induce constitutive activation. All 19 substitutions of D142 resulted in α1B-adrenergic mutants displaying different levels of constitutive activity with the D 142T mutation producing the highest levels (Scheer et al ,1997). Thus, point mutations for 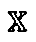 using all amino acids in the highly conserved 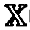 RY sequence in the second intracellular loop is a universal cassette mutation which will be used to activate G protein-coupled receptors in further embodiments of this invention.

### d. Universal Mutational Alignment

The C terminal region of the third intracellular loop adjacent to transmembrane section six has been shown to be important for G protein coupling. In the α1β-adrenergic receptor, substitution of any of the other 19 amino acids for alanine 293 resulted in constitutive activation. Substitution with either glutamic acid or lysine resulted in the highest levels of constitutive activation. The cassette is as follows:
EKKA 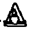 ( 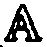 at position 293 is substituted in the α1β-adrenergic receptor)

The importance of this position is further substantiated by an analysis of a mutational data-base of the amino acid sequence alignment of all mutations which have been performed at this site and which have resulted in constitutive activation.

A list of amino acid substitutions corresponding to position 293 of the α1β-adrenergic receptor which result in constitutive activation of the receptor are as follows:
(1) α1β-adrenergic receptor A293 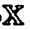 where 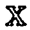 is all remaining amino acids (Kjelsberg et al. 1992).
(2) Alpha 2A-adrenergic receptor T373(A, C, E, K, F) (Ren et al. 1993).
(3) Beta 2A-adrenergic receptor L272(A,I,T) (Pei et al. 1994).
(4) LH/CG receptor A568V (Latronico et al. 1995).
(5) TSH receptor A623I (Parma et al. 1993).
(6) Serotonin 5HT2A C322K (Casey et al. 1996)
(7) Serotonin 5-HT2C S312K/F (Barker et al. 1994; Herrick-Davis, 1996).
(8) Platelet-activating factor receptor L231R (Parent et al. 1996).
(9) Muscarinic m2 receptor (T386A) (Blin et al. 1995)

Thus, it appears that changing the amino acid in this position to any other amino acid favors constitutive activation, but preferably the change is to a basic or acidic amino acid or another hydrophobic amino acid with differing side chains.

If the 4 amino acid α1β-adrenergic receptor sequence is examined, another key position appears to be the acidic glutamic acid residue before the double basic residues, as follows:
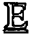 KKAA
Mutation of amino acids which align to this position also results in constitutive activation across a number of receptor types as listed below:
(1) Beta2-adrenergic receptor E268G (O'Dowd et al. 1988).
(2) Muscarinic M1 receptor E360A (Hogger et al. 1995).
(3) LH/CGR receptor D564G (Laue et al. 1995).
(4) TSH receptor D619(G) (Parma et al. 1993)

The amino acid motif represented by EKKAA is conserved to some extent in practically all G protein coupled receptors. For example, the double basic KK may be replaced by 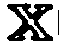 K or RR or KR or RK (where 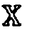 = any other amino acid).

Based on these alignment observations across a variety of different classes of G protein-coupled receptors, an overall mutational insertion cassette is disclosed which will have a general utility in constitutively activating G protein-coupled receptors. Due to the length differences between different G protein-coupled receptors, the numeric position (amino acid number) for cassette insertion will vary. However, the point of insertion is "positional" i.e., the point of insertion is at the junction of the third intracellular loop and transmembrane section six.
This cassette is:
X₁BBHyX₂

Where X₁ can be any amino acid, preferably G, A or K; where B is a basic amino acid; Hy is a hydrophobic amino acid, preferably A; X₂ is any amino acid, preferably K, R, E or a hydrophobic amino acid with a differing side chain to the original hydrophobic amino acid in that position. As further embodiments of this invention, this universal mutational cassette is most preferably utilized at the junction of the third intracellular loop and transmembrane section six to constitutively activate G protein-coupled receptors.

### 2. Tyrosine Kinase Receptors

### a. General Methods

A number, of methods set forth below will be used to constitutively activate tyrosine kinase receptors in further embodiments of this invention. Transforming cells with the endogenous oncogene (e.g. src gene product) has been shown to result in increased intracellular phosphorylation and activation of the IGF-I receptor. Also, point mutations in the kinase domains of these receptors have led to constitutive activation. Thus, by transferring a constitutively active kinase domain from one tyrosine kinases receptor to another, which is not active, it is believed will result in constitutive activation of that receptor.

Many tyrosine kinase receptors require dimerization to be active. Point mutations around the transmembrane regions of these receptors, have been shown to lead to ligand-independent dimerization and cellular activation. This has been particularly relevant in the constitutive activation of the fibroblast growth factor 2 receptor by a mutation of C332 to Y which led to increased receptor dimerization by the formation of an intermolecular disulfide bond (Neilson et al. 1995) which, in turn, results in Crouzon syndrome. Thus, it is believed that mutations in the tyrosine kinase receptors in this region, especially at cysteine residues, will result in constitutive activation of other receptors and such mutation will be used as a further embodiment of this invention.

From analysis of mutational deletions, it is thought that the extracellular α-subunit of the insulin receptor exerts an inhibitory effect on the intracellular kinase activity. Deletion of N281, in the insulin receptor α-subunit resulted in constitutive activation which is thought to be due to removal of the inhibitory effect of the α-subunit on the intracellular kinase activity (Desbois et al. 1996). Similar deletions in tyrosine kinase receptors with structures similar to insulin will be used in a further embodiment of this invention.

The met gene, encoding the tyrosine kinase receptor for hepatocyte growth factor, is a potentially harmful oncogene expressed in a significant fraction of human cancers. The cytoplasmic domain truncated immediately below the transmembrane region was shown to acquire constitutive activity (Zhen et al. 1994). Thus, it appears that the extracellular regions of these receptor tyrosine kinases maintain an inhibitory effect on the ability of the intracellular kinase domains to become activated. Thus, point mutations in the extracellular regions of these receptors will be used in another embodiment of this invention to remove the inhibitory effects on the intracellular kinase domains as has been shown for the insulin receptor above.

Furthermore, a naturally occurring spliced variant of the RON tyrosine kinase has been shown to result in constitutive activation (Collesi et al. 1996). This transcript differs from the full length protein by an in-frame deletion of 49 amino acids in the β-chain extracellular domain. Thus, deletions of a similar size in related tyrosine kinase receptors will be used in other embodiments of this invention as a method to constitutively activate these receptors. Similarly, a naturally occurring point mutation in the c-kit receptor has resulted in deletion of seven amino acids in the juxtamembrane domain which resulted in constitutive activation (Tsujimura et al. 1996). The juxtamembrane sequence of TQLPYDH was also found to be present in the mouse CSF-1 receptor and the α-PDGF receptor. Thus, deletion of this sequence in these receptors and other homologous receptors will be used to constitutively activate these tyrosine kinases in the practice of this invention.

### b. The Extracellular Binding Domain

Receptor oligomerization is a universal phenomenon among growth factor receptors. It may be induced by monomeric ligands, such as EGF, that induce conformational changes resulting in receptor-receptor interactions, or bivalent ligands, such as PDGF and CSF-1, that mediate dimerization of neighboring receptors. Oligomerized growth factor receptors possess elevated protein tyrosine kinase activity and enhanced ligand binding affinity. Thus, the extracellular domain of the tyrosine kinase receptor family will be exploited in additional embodiments of this invention for the purpose of constitutive activation by enhancing oligomerization. This will be achieved by using deletional cassettes which will delete one or more cysteine residues from the extracellular domain which will leave an odd cysteine to form intermolecular disulfide bonds resulting in oligomerization and receptor activation. The effectiveness of this mechanism is exemplified by the RON receptor. A mutated form of the RON gene product is encoded by a transcript differing from the full-length RON mRNA by an in-frame deletion of 49 amino acids in the β-chain extracellular domain (Collesi et al. 1996). The mutated receptor is constitutively activated by disulfide-linked intracellular oligomerization because it contains an uneven number of cysteine residues. The mutated RON receptor is formed by alternate splicing of a true cassette exon of 174 bp encoding a short segment of the transmembrane domain. This results in the loss of three cysteine residues, leaving an uneven number which can now homodimerize by disulfide exchange.

### c. Transmembrane Domain & Mutational Cassettes

All the members of the receptor tyrosine kinase family have a single spanning transmembrane domain. This common feature of the family can be exploited to constitutively activate the receptors. An example of the effectiveness of this approach is provided by the observation that substitution of the insulin receptor transmembrane domain with activated Neu (E664) results in insulin-independent receptor dimerization and activation (Yamada et al. 1992; Cheatham et al. 1993). Thus, in an alternate embodiment of this invention, mutated transmembrane cassettes will be transferred across subclasses of the tyrosine kinase family of receptors to activate receptors of other subclasses.

In addition, point mutations in the transmembrane domain of the fibroblast growth factor 3 receptor (FGFR3) have resulted in constitutive activation. Achondroplasia is the most common genetic form of dwarfism. This has been shown to result from a G to R substitution in the transmembrane domain of the fibroblast growth factor 3 receptor. More importantly, by substituting the transmembrane domain of the Neu receptor tyrosine kinase with the transmembrane domains of the wild type and mutant FGFR3, the R380 mutation in the FGHFR3 is shown to activate both kinase and transforming activities of this chimeric receptor. Residues with side chains capable of participating in hydrogen bond formation, including E, D, and to a lesser extent, Q, H and K, were able to substitute for the activating R380 mutation.

A mutational transmembrane cassette (FGF3R; G380R) is as follows:
VYAGILSY 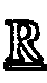 RVGFFLFILVVAAVTLC
or, VYAGILSY 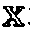 XGFFLFILVVAAVTLC (where 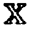 = E, D, Q, H or K)
Also, it has been shown that the mutation of V664 to E provides for the oncogenic activity of Neu receptor tyrosine kinase.

A further mutational transmembrane cassette (Neu/erb-2 receptor) is:
VTFIIATV 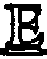 GVLLFLILVVVVGILI
The underlined sequence is a motif found in many receptor tyrosine kinase transmembrane domains (Sternberg et al. 1990). It is thought that these mutations may mediate dimerization of the receptor. Consistent with this model of transmembrane-mediated dimerization is the observation that substitution of the insulin receptor transmembrane domain with activated Neu (E664) results in insulin-independent receptor dimerization and activation (Yamada et al. 1992; Cheatham et al. 1993). As further embodiments of the present invention, chimeric receptors as indicated will be made, and additional embodiments will introduce the transmembrane mutational cassettes to activate other tyrosine kinase receptors.

### d. The Juxtamembrane Domain

The transmembrane domain of receptor tyrosine kinases is separated from the cytoplasmic, catalytic domain, by juxtamembrane sequences that are divergent between receptor subclasses but conserved between members of the same subclass. A great deal of evidence suggests that this domain is involved in modulation of receptor functions by heterologous stimuli, a process termed receptor transmodulation. For example, activation of PDGF or bombesin receptors by their respective ligands leads to the abolishment of high affinity EGF binding sites in a single cell. This effect is triggered by phosphorylation of residues in the juxtamembrane domain. Thus, it appears that the tyrosine kinase receptors negatively regulate themselves by phosphorylation of residues in the juxtamembrane domain. The importance of this domain is further demonstrated by the ability to constitutively activate the c-kit receptor by deletion of seven amino acids at the juxtamembrane domain (Tsujimura et al. 1996). The deleted segment corresponded to TQLPYDH (codons 573 to 579). Interestingly, the seven amino acid sequence is widely conserved in all of mouse, rat, cat, cattle, human and chicken KITs. Furthermore, the four amino acid sequence, QLPY, within the seven amino acid sequence is entirely conserved in type II tyrosine kinase receptors such as KIT and PDGF. Therefore, it is probable that the deleted region of KIT may have an important role in the structure and function of type III receptor tyrosine kinases. It is thought that this domain, particularly Y569, may also be involved in ligand-induced receptor internalization (Myles et al. 1994). Thus, as additional embodiments of this invention, deletional mutations of the juxtamembrane domain in this vicinity will be utilized to remove the negative overriding effect of this domain to constitutively activate a variety of known and orphan receptor tyrosine kinases.

### e. The Carboxy-terminal Tail

The carboxy-terminal tail sequences are among the most divergent between all known receptor tyrosine kinases. The carboxy terminal tail of the EGF receptor may possess enough length and flexibility to interact with the substrate binding sites of the protein tyrosine kinase region and modulate its capacity to interact with exogenous substrates. Consistent with this model, cells expressing mutant EGF receptors with altered autophosphorylation sites were mitogenically responsive to lower doses of EGF than cells expressing similar levels of wild-type receptors. Deletions in the carboxy-terminal tail had a similar effect on transformation: they potentiated oncogenic capacity and increased the host range but did not provide a major oncogenic lesion. Similar experiments in other cell systems and with other receptors such as that for CSF-1, support the notion that the carboxy-terminal tail sequences exert negative control on receptor tyrosine kinase signalling functions. Thus, carboxy terminal deletion cassettes will be used to constitutively activate tyrosine kinase receptors in additional embodiments of this invention.

### B. Anti-Peptide Antibody Stimulation of Target Receptors

### 1. G Protein-Coypled Receptors

Antibodies directed against certain sites within the extracellular loops of G protein-coupled receptors have been shown to induce constitutive activation, presumably by their ability to functionally simulate ligand binding. Alla and colleagues have described the ability of antibodies directed against the N-terminal portion of extracellular loop two of the bradykinin B2 receptor to increase intracellular responses in the absence of ligand (see Alla et al. 1996). Thus, antibodies raised against synthetic peptide sequences directed against the N-terminal portion of the extracellular loop will be used to constitutively activate G protein-coupled receptors. Such Antibodies can be raised, for example, in rabbits using standard techniques and purified by affinity chromatography.

### 2. Tyrosine Kinase Receptors

Antibodies generated against the extracellular region of the erB2/Her2 receptor strongly induce tyrosine phosphorylation of 180-185 kDa proteins including the Her2, Her3, and Her4 receptors, when expressed on the surface of breast cancer cells. Interestingly, the antibodies do not cross react with Her3 or Her4. Thus, antibodies raised against this portion of tyrosine kinase receptors will be used to create further constitutively activated receptors as further embodiments of this invention.

### C. Overexpression Of Receptors and G Proteins

### 1. G Protein-Coupled Receptors

Overexpression of seven transmembrane G protein-coupled receptors has been shown to result in constitutive activation. Agonist-independent properties of the human β2-adrenergic receptor have been studied in the baculovirus expression system in Sf9 cells (Chidiac et al. 1994). The increase in cAMP production was proportional to the number of receptors expressed. Thus, overexpression of these receptors in the baculovirus system leads to constitutive activation.

### 2. G Proteins

As noted earlier, G protein-coupled receptors exist in an equilibrium between an inactive state and an active state which couples to a G protein mediated transduction pathway. While the transition between the two states involves a conformational change about which little is known, the endogenous ligand (agonist) for the receptor stabilizes the active conformation, thereby shifting the equilibrium towards the active state. As with any system in equilibrium, the equilibrium can be shifted in favor of one side or another by external forces which supply or remove one of the components of the equilibrium. For G protein-coupled receptors, the equilibrium can be shifted towards the active state by decreasing the concentration of the active and non-G protein bound form of the receptor available to participate in the equilibrium. This effect has been demonstrated for the m1, m3, and m5 muscarinic receptors (Burstein et al. 1995). Increasing the concentration of the G proteins with which the receptor binds shifts the equilibrium so that the proportion of receptors in the active state increases. The G protein is preferably a promiscuous G protein; that is, one which couples with a wide range of receptor classes (e.g. G_{α15}). This form of constitutive activation will be applied to diverse G protein-coupled receptors to produce constitutively activated receptors.

### 3. Tyrosine Kinase Receptors

Overexpression of the erb-2 product has resulted in constitutive activation (Lonardo et al. 1990). Thus, overexpression of tyrosine kinase receptors can be used as a method to constitutively activate these receptors.

### II. TRANSFECTION OF CONSTITUTIVELY ACTIVE RECEPTOR INTO IN-VITRO EXPRESSION SYSTEM

As mentioned earlier, in situations where an endogenous constitutively activated receptor is not available, once a constitutively active form of a receptor is created through the manipulation of the genetic sequence coding for a receptor, the next step in the method of this invention is to place the coding sequence in an expression system which will produce the specified receptor protein. As a test for the success of the expression system, and before looking for cellular mediated receptor effects, it is desirable to confirm that the receptor is being properly expressed.

### A. Standard Techniques

A number of methods are available and well known among those knowledgeable in the art for introducing receptor cDNAs into cultured mammalian cells, including: calcium phosphate or DEAE-dextran-mediated transfection, polybrene transfection, protoplast fusion, electroporation, liposomes and direct injection into the cell nucleus. The most widely used method is transfection with calcium phosphate because of its high efficiency. Briefly, the method involves: harvesting exponentially growing cells by trypsinization and re-plating them at a density of 1x10⁵ - 2x10⁵ cells/culture dish in the appropriate serum-containing medium. A number of cell types can be used, including NIH-3T3, HeLa S3, COS and CHO etc. The cultures are then incubated for 20-24 hours at 37° C at an atmosphere of 5-7% CO₂. A calcium phosphate-DNA coprecipitate is prepared for each monolayer of cells; the receptor cDNA is dissolved in 0.1M Tris EDTA buffer at a concentration of approximately 40µg/ml, 220µl of this mixture is combined with 250µl of 2X HEPES-buffered saline in a sterile plastic tube (e.g. Falcon 2509) and a total of 31µl of 2M calcium chloride is added over 30 seconds. This mixture is incubated for 30 minutes at room temperature and then transferred to the medium above the monolayer of cells. The transfected cells are then incubated for 24 hours at 37° C in an atmosphere of 5-7% CO₂ . The medium and precipitate are then removed by aspiration, the cell layer is washed once with phosphate-buffered saline and 5ml of pre-warmed complete growth medium is added prior to incubating the cells for a further 24 hours at 37° C. The cells are then replated in the appropriate selective medium for the isolation of stable transformants. Molecular Cloning, A Laboratory Manual Vol 3, 16.30, Editors Sambrook, Fritsch and Maniatis, 1989, a standard reference work in the art, sets out detailed explanations of this and other methods of transfection.

### B. Confirmation of Constitutively Activated Receptors With Phosphorylation Assay

Receptor phosphorylation will be used as a first means of measuring constitutive activity in the expression system because only activated receptors are substrates for receptor kinases. This method has been used to measure the activated state of β-adrenergic receptors (Samama et al. 1993). Briefly, purified receptor preparations are incubated at 30°C for 20 min with β-adrenergic receptors (30 nM) in 20 mM Tris.HCl, pH 8.0, 2 mM EDTA, 10 mM MgCl, 1 mM dithiothreitol, 100 µM [γ-³²P]ATP (∼2000 cpm/pmol), in the absence and presence of the relevant drugs (for orphan receptors the difference in basal phosphorylation level between constitutively active and non-active receptor will be used). At the appropriate times the reactions are stopped with an equal volume of 2X SDS sample-loading buffer (8 % SDS, 25 mM Tris.HCl, pH 6.5, 10% glycerol, 5% mercaptoethanol, and 0.005% bromophenol blue) and are then electrophoresed on 10% SDS-polyacrylamide gels. Phosphorylation stoichiometries are then determined by excising and counting the receptor bands by using a phosphoImager.

### III. USE OF SECOND MESSENGER ASSAYS TO DETECT COMPOUND EFFICACY

Once expression and activity of the constitutively activated receptors have been confirmed, in order to study the compound efficacy of candidate compounds on the receptor, a method to detect the receptor-directed cellular response is most prefered. Several assay methods well known to those skilled in the art may be used with little modification. Because the invention disclosed herein allows the skilled artisan to screen large numbers of candidate compounds (e.g., chemical libraries) against the constitutively activated receptors, assay methods which lend themselves to high throughput screening are particularly advantageous. Examples of different assay methods that will be used are set forth below. These examples should be considered illustrative (and not limiting) of the type of assays which can be employed in the method of this invention. Different receptor types require different assays, and for any given receptor, one assay may be more appropriate than another. These choices are readily determined without undue experimentation and are well appreciated and understood by one skilled in the art.

### A. G Protein-Coupled Receptors

### 1. GTP Membrane Binding & GTPase activity

It has been reported that [³⁵S]GTPγS can be used to monitor G-protein coupling to membranes in the absence and presence of ligand (Traynor and Nahorski, 1995). This assay is performed on membranes which have the expressed receptor. Briefly, membranes (100-200 µg of protein) are incubated with binding buffer (20 mM HEPES, pH 7.4, 100 mM NaCl, and 10 mM MgCl.6H₂0) containing [³⁵S]GTPγS (80 pM), GDP (3 µM), and the small molecule drug (10-20 µM). The reported volume for performing this assay was 1 ml, but volumes ranging from 0.3-1 ml are useful. This is incubated for 60 min at 30°C. Bound and free [³⁵S]GTPγS can then be separated by vacuum filtration through GF/B filters and quantified by liquid scintillation counting. Inverse agonists are detected by their ability to reduce the scintillation signal.

### a. GTP membrane binding for a scintillation proximity assay

A high-throughput screening assay can be used to measure the GTP binding to receptors using a scintillation proximity assay. Flash plates or scintistrips can be used which have scintillant coated on the wells. The binding assay can be performed as described above. However, when the membranes are centrifuged to the bottom of the wells, there is preferably need for separation of bound and "free" [³⁵S]GTPγS because the bound [³⁵S]GTPγS on the third intracellular loop will be in close proximity to the scintillant which will result in a signal.

Another way of performing the assay is to immobilize the membranes containing the expressed receptor onto flash plates. Briefly, the plates are coated with polylysine. The positively charged polylysine binds to the negatively charged phosphate groups and immobilizes the membranes. Any bound [³⁵S]GTPγS is then in close proximity to the scintillant and activates the scintillant, producing a signal. The assay can then be used to detect chemical compounds which inhibit this signal, which compounds are inverse agonists.

Another way of performing the assay will be to immobilize the membranes using Con A. Most seven transmembrane receptors contain sugar groups expressed on the extracellular regions of the receptor. Con A will bind to the sugar groups expressed on the extracellular side of the membrane and immobilize the membranes. The bound [³⁵S]GTPγS can then be detected by scintillation proximity as before.

### b. GTPase activity

GTPase activity can also be measured by incubating the membranes containing the expressed receptor with [γ³²P]GTP. The released [γ³²P]Pi is then detected in the supernatant by scintillation counting (Hasegawa et al. 1996). Inverse agonists are detected by their ability to reduce the signal and agonists by their ability to increase the signal.

### 2 Adenylate Cyclase and Cyclic AMP (cAMP) Assays

Adenylate cyclase assays can be used to assess the efficacy of drugs interacting with constitutively active receptors. Briefly, the reaction medium contains phosphocreatine 5mM; creatine kinase 10 U/ml; BSA 0.04 % ; Tris HCl 50 mM Ph 7.4; MgCl₂ 5 mM; EDTA 0.25 mM; RO20-1724 0.12 mM; ATP 0.1 mM; GTP 0.1 mM [α-³²P]ATP between 1.5 and 2.5 µCi/tube; sucrose final concentration 65 mM. Assays are initiated by the addition of membrane suspension (∼50 to 75 µg protein/tube) and the assay mixture incubated at 31°C for 1 h. (see Maenhaut et al. 1990 & Salamon et al 1974). Intracellular and extracellular cAMP can be determined using a standard cAMP radioimmunoassay or by using cAMP binding protein according to standard art methods.

### 3. Whole Cell Second Messenger Reporter Systems

Promoters on genes drive the expression of the proteins which that particular gene codes for. Cyclic AMP drives gene expression by promoting the binding of a cAMP-responsive DNA binding protein or transcription factor (CREB) which then binds to the promoter at specific sites called cAMP response elements and drives the expression of the gene. Reporter systems will be constructed which have a promoter containing multiple cAMP response elements before the reporter gene which can be β-galactosidase or luciferase, for example. Thus, an activated receptor causes the accumulation of cAMP (if it is coupled to a stimulatory G-protein) which then activates the gene and expression of the reporter protein. The reporter protein such as β-galactosidase or luciferase can then be detected using standard biochemical assays (Chen et al. 1995). Thus, a stable cell line will be constructed which has the cAMP-reporter construct. The activated receptor will then be transfected into this cell line which will activate the reporter gene expression. This assay system will then be used in the presence of small molecules from a chemical library to find compounds which inhibit the reporter gene expression by interacting with the receptor; such inhibitors are inverse agonists identified by the method of this invention.

### 4. Intracellular Calcium Detection Using Calcium Sensitive Dyes

The activation of receptors on the cell surface often leads to an increase in intracellular "free" calcium levels. This increase in intracellular calcium can be detected in whole cell assays using calcium-sensitive dyes such as fura 2. A decrease in this signal caused by a chemical compound indicates inverse agonist activity.

### 5. Immediate Early Gene Induction

Early gene induction and expression is a downstream event of receptor activation. Activation and expression of transcription factors such as c-fos and CREB will be used as a, means to monitor receptor activation and inhibition by drugs. The expressed CREB and c-fos can be detected by anti CREB and c-fos antibodies. These antibodies can be further detected by standard methods (e.g. anti rabbit horse-radish peroxidase antibodies with conventional peroxidase detection methods), with a decrease in the signal caused by a chemical compound indicating inverse agonist activity.

### 6. Cell Proliferation

Activation of G protein coupled receptors and tyrosine kinase receptors often results in cell proliferation. The ability of the cell to proliferate can be measured by the uptake of [³H] thymidine into the DNA. Chemical compounds which block this proliferation can then be detected by a decrease in thymidine incorporation. The incorporation of bromo-deoxyuridine (BRDU) into the DNA can also be used as a measure of cell proliferation and receptor activation. The incorporated BRDU can be detected by scintillation counting or by anti-BRDU antibodies according to well known methods to determine compound-induced effects.

### B. Tyrosine Kinase Receptors

Whole-cell second messenger reporter systems and intracellular calcium detection using calcium-sensitive dyes as described in sections C.1.c., C.1.d., and C.1.e. above will also be used to assay the tyrosine kinase activity.

### IV. SCREENING OF CANDIDATE COMPOUNDS WITH CONSTITUTIVELY ACTIVATED RECEPTOR SYSTEMS

The method of this invention is well suited to modern techniques of high throughput screening (HTS) of, most preferably, large chemical libraries. Generally, when searching for potential drug candidates, what is desired is a compound which will have a high bioavailability. Recent advances in combinatorial chemical synthesis have produced large libraries of small organic compounds which have the potential to meet the bioavailability requirements. Since, once the method of this invention is known and appreciated, the technology exists for producing and assaying large numbers of constitutively activated receptor expression systems, high throughput screening of, for example, such chemical libraries or peptide libraries (e.g., in 96 well plate format) is straightforward. Therefore, application of the method of this invention is very useful as a straightforward manner to discover useful therapeutic compounds. A few examples of how such assays and screening are carried out illustrate the general approach, but are not intended to limit the scope of this invention.

### A. Scintillation proximity assay (SPA) for cAMP

Constitutively active G-protein coupled receptors will be screened in a high throughput manner by measuring the formation of cAMP by scintillation proximity assay. One way of achieving this is to link an anti-CAMP antibody to scintillation proximity beads which are coated with scintillant. Thus, when radiolabeled CAMP is bound by the anti-cAMP antibody which is attached to the bead it comes into close proximity with the scintillant and activates it. Unlike conventional binding assays, this assay does not require any separation of bound and "free" cAMP as the signal is purely detected by the proximity of the molecules in solution. As such this assay provides a means for high-throughput drug screening for small molecule inverse agonists to constitutively active G-protein coupled receptors.

One example of how to perform this assay is as follows: briefly, membranes (5-10 µg total protein) expressing the receptor of interest are incubated with 0.12 mM ATP, 1-2 x 10⁶ cpm/assay tube of [α-³²P]ATP, 0.10 mM cAMP, 53 µM GTP, 2.7 mM phospho(enol)pyruvate, 1.0 IU of myokinase, 0.2 IU of pyruvate kinase, 4 µg/ml benzamidine, 2 µg/ml soybean trypsin inhibitor, 2 µg/ml leupeptin and varying concentrations of test compounds in a total volume of 50-300 µl. Samples are then incubated at 37°C for 15 or 30 min, and the reaction is terminated by addition of 0.5-1 ml of a cold solution containing 0.3 mM cAMP, 20,000 cpm of [³H]cAMP, and 0.4 mM ATP (Chidiac et al. 1994). The constitutively active receptors promote the formation of [³²P]cAMP which is then specifically captured by the anti-cAMP antibody which is linked to the scintillation bead. This results in a signal after scintillation counting. Any test compounds which have inverse agonist activity at the receptor will decrease the amount of [³²P]cAMP formed which in turn will be measured as a decrease in the signal after scintillation counting. Traditionally, the [³²P]cAMP was laboriously separated for measurement by Dowex gel chromatography. The scintillation proximity assay allows for rapid detection of the cAMP without need for a prior separation technique, allowing the adaption of this assay for high throughput screening of compound libraries for inverse agonists to constitutively active known and orphan G protein-coupled receptors.

Another adaption of this assay will be to capture the [³²P]cAMP by a cAMP-dependent binding protein instead of the anti cAMP antibody. A cAMP-dependent protein kinase can be obtained from Sigma Chemical Co. (St. Louis, MO) for this use. Briefly, the cAMP-dependent protein kinase which is purified from porcine heart will be coupled to the scintillation beads. The [³²P]cAMP which is formed can then be captured by the cAMP-dependent protein kinase which is coupled to the SPA bead. Amersham manufactures a commercial kit to measure cAMP by scintillation proximity (Hortaon & Baxendale, 1995) which can be employed in the practice of this invention.

Another alternative to the above methods will be to use the scintillation proximity assay as a competitive binding assay to measures the quantity of unlabeled cAMP which is produced by the receptor system. Briefly, the conditions will be similar except labeled [³²P]cAMP will not be included. The constitutively active receptor will produce cAMP. This cAMP can then be measured by the cAMP scintillation proximity assay in the presence of a trace amount of radiolabeled cAMP. Thus, the more cAMP is present in the sample the more unlabelled cAMP will be bound by the antibody or cAMP binding protein on the scintillation beads and the less radiolabeled cAMP will be left in the proximity of the beads to give a signal. Samples can then be read against a standard curve of cAMP to estimate how much cAMP has been produced.

### B. Measurement of cAMP by enzyme-linked immunoassay

The released cAMP which is formed by the constitutively active receptor can also be detected by enzyme-linked immunoassay (Horton & Baxendale, 1995).

### C. Measurement of cAMP by cAMP-dependent protein kinase A

Protein kinase A utilizes cAMP as a co-factor to phosphorylate substrates. Thus, the formation of cAMP will also be detected by monitoring the ability of cAMP-dependent protein kinase A to phosphorylate a substrate such as casein.

[γ-³²P]ATP + casein will be converted in the presence of protein kinase A and cAMP to [³²P]casein and ADP. The phosphorylated casein can be isolated on filter discs and the protein bound ³²P is measured. The protein kinase A enzyme becomes more active as more cAMP is present resulting in a greater degree of phosphorylation of the substrate casein. The quantity of cAMP produced can be estimated relative to a standard curve of known cAMP concentrations. This assay will also be adapted for scintillation proximity by linking the substrate e.g. casein to SPA beads. Thus, as more [³²P]casein is produced more signal will be detected by proximity to the beads which can be used to calculate the concentration of cAMP relative to a standard curve.

### D. The RAS scintillation proximity assay

The Ras oncogene encodes a guanine nucleotide binding protein of MW 21,000. The protein acts as a molecular switch in a signal transduction pathway. In its GDP form it is inactive, whereas in the GTP form it interacts with one or more effector molecules to send a signal. The Ras oncogene has been implicated in many human cancers. Furthermore, both tyrosine kinase receptors and G-protein coupled receptors have been shown to utilize the Ras signalling pathway (Sharif et al. 1994). An assay has been described which measures the interaction of Ras with another effector molecule, called nerofibromin (NF1) using scintillation proximity assay (Skinner et al. 1994). Thus, membrane extracts having both expressed tyrosine kinase and G protein-coupled receptors will be used to activate recombinant Ras protein in this system. Ras is activated when it binds radiolabeled [³H]-GTP which can be generated by nucleotide exchanged in the presence of a GTP-regenerating system. A SPA signal is then obtained when radiolabeled Ras is mixed with NF1 fused with glutathione S-transferase (GST), anti GST and protein A-coated SPA beads. Thus, as Ras is activated by the constitutively active receptor more [³H] GTP will be bound to Ras and as such more Ras complexes with NF1 which will result in a SPA signal. Thus, according to the method of this invention, small molecule inverse agonists will be detected by a decrease in this signal.

The oncogenic Ras proteins, e.g. L-61 Ras, bind to Ras GAP (another effector molecule) but their GTPase activity is not stimulated (normally upon binding to GAP, GTP is hydrolyzed to GDP). This apparently results in a constitutive mitogenic or transforming signal. Thus, oncogenic Ras will be used as a high affinity GTP binding protein to measure GTP levels. For example, the amount of GTP which is hydrolyzed by a G protein coupled to a constitutively active receptor will be measured by Ras binding. One example of how to do this will be to couple the oncogenic form of Ras to an SPA bead. The more radiolabeled GTP which is hydrolyzed by the constitutively active receptor, the less will be bound by Ras coupled to the SPA bead. Thus, the more constitutively active the receptor, the less SPA signal detected. As such, in this system, small molecule inverse agonists will be detected by an increase in SPA signal as they will prevent the GTPase activity of the G-protein-coupled receptor system. Thus, scintillation proximity assays utilizing the oncogenic Ras binding protein will be used to measure the index of GTP hydrolysis by G-protein coupled receptors providing a means for high-throughput screening for small molecule inverse agonists.

### V. PREFERRED USES OF THE INVENTION

The present invention significantly accelerates the discovery of therapeutic molecules of great importance in the treatment of human disease. The most significant feature of this invention is that modulators of receptor function can be identified without any prior knowledge of an endogenous or synthetic ligand for the receptor. This is particularly important with orphan receptors, and the method of this invention is particularly suited for use with orphan receptors. Not only is the endogenous ligand not known for orphan receptors, but constitutively active mutations have not usually been identified. Appropriate high throughput screening of compound libraries will permit the identification of both receptor agonists and inverse agonists.

In addition to the ability of the method of this invention to capitalize on existing and emerging genetic information on orphan receptors, drugs developed using this technology at known receptors are intended to possess distinct therapeutic advantages over drugs developed using traditional ligand-based screens. Ligand-based screens only identify compounds which act to "antagonize" the action of the ligand on the receptor and thereby prevent ligand-induced receptor activation. Such compounds, or antagonists, do not block ligand-independent receptor activation and the subsequent cellular response. On the other hand, inverse agonist drugs acting at constitutively activated receptors, identified using this invention, block both ligand-dependent and ligand-independent receptor activation. This property of compounds (drugs) identified by the method of this invention is extremely relevant to the growing number of diseases which have been linked to constitutively active G protein coupled receptors, since traditional antagonists will not block such activity.

As noted, inverse agonists discovered using the method of this invention have utility as therapeutic agents for the treatment of various diseases and disorders which are characterized by constitutive activation of a receptor. The routine application of the methods disclosed herein allow a practitioner of ordinary skill, without undue experimentation, to directly identify candidate compounds which have, most preferably, the requisite inverse agonist activity, e.g. by utilizing conventional high-throughput screening of chemically diverse compound libraries which have been constructed by methods which are known to those skilled in the art. Thus, such inverse agonists are directly identified by direct use of the methods disclosed herein, but which could not be readily identified as such by other methods, e.g. the conventional method which first requires identification of the endogenous ligand. Such directly identified inverse agonists are useful for treatment of disorders or diseases characterized by constitutive activation of a receptor. Appropriate methods of administering the compounds of the invention as pharmaceutical compositions, including dosage and formulation, will be apparent to those skilled in the art. The mode of administration, dosage, and formulation of such pharmaceutical compositions will depend upon the medical condition being treated, as well as the general health of the patient, these factors being within the purview of the skilled artisan.

For instance, for Graves' disease, candidate compounds can be directly identified with this invention to identify those which directly block the constitutively active TSH receptor in the thyroid, thereby negating or reducing the need for surgical removal or radioiodine destruction. Thus, a basis for a newer and more effective therapeutic strategy is provided.. In male precocious puberty, the severity of the puberty seems to be directly related to the degree of constitutive LH receptor activity. The method of this invention is directly applicable to the direct identification of an inverse agonist to the constitutively active LH receptor. The invention provides a similar approach for directly identifying compounds which are inverse agonists for the treatment of the constitutively activated receptor diseases discussed earlier in this application.

Another important application of the invention will be in identifying an inverse agonist to the gammaherpes virus transmitted constitutively active G protein-coupled receptor which causes cancerous cell growth in Kaposi's sarcoma and is frequently seen in HIV infected individuals. Since the endogenous ligand for the transmitted receptor is unknown, this is an example of the utility of this invention providing a method to identify an inverse agonist to an orphan receptor. Kaposi's sarcoma provides a good example of where knowledge of an agonist or even of an antagonist will not yield a compound which will reverse the cancer-causing activated receptor. However, the method of this invention is designed to screen for exactly the compound necessary, an inverse agonist.

Two notable neuropsychiatric diseases associated with constitutively activated receptors are schizophrenia (overexpression of dopamine D4 receptors) and major depression (overexpression of serotonin 5-HT_{2A} receptors). Inverse agonists to the active receptors promise to be a beneficial treatment. The same approach of identifying inverse agonists with the method of this invention will be used with the constitutively active tyrosine kinase disease related receptors as well as with other types of receptors. It is further to be expected that as the function of more orphan receptors become known, many more cases of human disease associated with constitutively activated forms of those receptors will be recognized.

It should be evident that the method of this invention is also useful for screening known drugs to determine if they may exhibit activity as inverse agonists to both known and orphan receptors. In addition, the methods of constitutively activating receptors described above, will be used to activate receptors for use in generating transgenic and/or gene-targeted animals which express the constitutively activated receptor. These non-human animals will be used to determine the physiological effect of the altered receptor function both to determine (in the case of orphan receptors) the physiological function of the receptor and to discover or characterize the physiological effect of any compound which acts at the receptor. Such additional applications of the disclosures of this invention are considered within the teaching and scope of this invention.

Different embodiments of the invention will consist of different constitutively activated receptors, different expression systems, and different assays. Those skilled in the art will understand which receptors to use with which expression systems and assay methods. All are considered within the scope of the teaching of this invention. In addition, those skilled in the art will recognize that various modifications, additions, substitutions, and variations to the illustrative examples set forth herein can be made within the scope of the invention.

### EXAMPLE 1

### Non-Endogenous Constitutively Activated Human Serotonin 5-HT_{2C}-G Protein-Coupled Receptor By Alignment and Point Mutation

In 1990 Cotecchia et al. demonstrated that the hamster α_{1B}-adrenergic receptor could be constitutively activated by mutation of amino acid 293 which is in the third intracellular loop near the transmembrane domain. Subsequently, as noted earlier, Kjelsberg et al. demonstrated that all 19 possible amino acid substitutions at position 293 confer constitutive activity to varying degrees. Recently, Teitler et al. has demonstrated that the rat serotonin 5-HT_{2A} and 5-HT_{2C} receptors may be constitutively activated if the amino acid positions in those receptors analogous to position 293 in the α_{1B}-adrenergic are mutated (322 in the 5HT_{2A} and 312 in the 5-HT_{2C}). While the sequence for the human serotonin 5-HT_{2A} and 5-HT_{2C} receptors is known, to date no evidence has been published that has shown that the human receptor can be constitutively activated.

The human 5-HT_{2C} serotonin receptor has now been constitutively activated by mutating the amino acid at the analogous position (310) in the human 5-HT_{2C} receptor from serine to lysine (S310K). Several methods have been used to verify the constitutive activation of the human 5-HT_{2C} receptor. One indication of constitutive activation is that the constitutively actived receptor has a higher affinity for the endogenous ligand than does the native receptor.

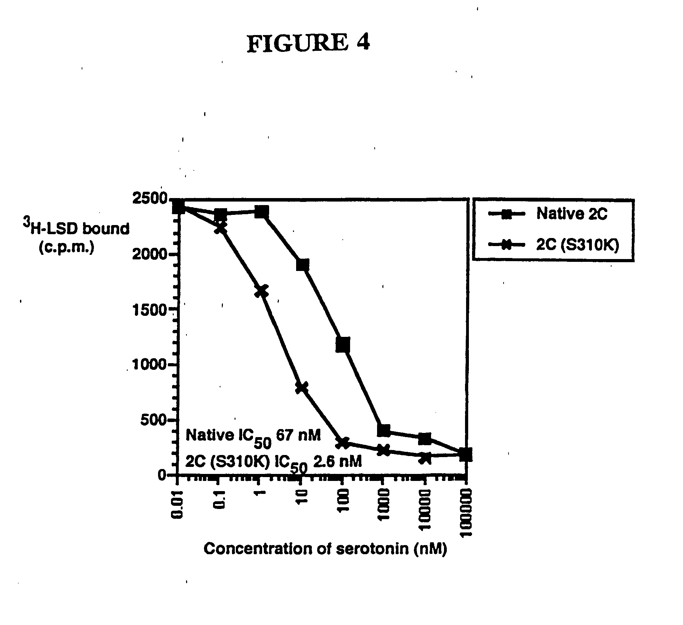

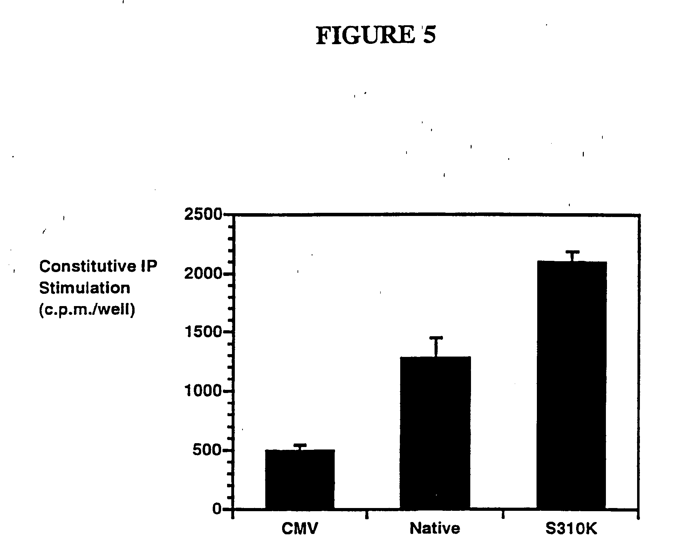

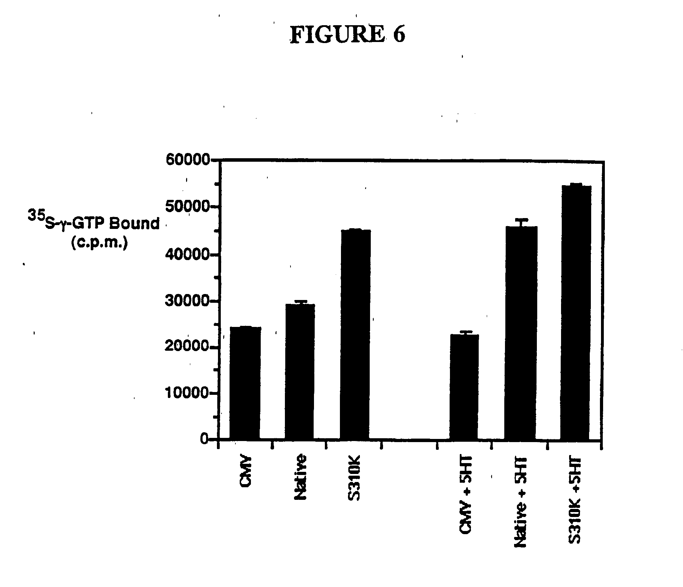

Figure 4 shows that the mutated human 5-HT_{2C} receptor has an enhanced affinity for serotonin over that of the native receptor. Another indication of constitutive activation is the enhanced activity observed in the second messenger pathway in the absence of endogenous ligands. Figure 5 shows that the mutated receptor produces enhanced intracellular accumulation of inositol triphosphate relative to the endogenous receptor. Finally, in Figure 6, constitutive activation of the 5-HT_{2C} receptor is shown by the mutated receptor's enhanced binding of [³⁵S]GTγS.

### EXAMPLE 2

### GTP Membrane Binding Scintillation Proximity Assay And Its Use In Identifying Constitutively Actived Receptors

When a G protein-coupled receptor is in its active state, either as a result of ligand binding or constitutive activation, the receptor binds to a membrane protein (called a G protein) and stimulates the binding of GTP to the G protein. The trimeric G protein-receptor complex acts as a GTPase and slowly hydrolyzes the GTP to GDP, at which point the receptor normally is deactivated. However, constitutively actived receptors continue to exchange GDP for GTP. The non-hydrolyzable GTP analog, [³⁵S]GTP-γS, can be utilized to demonstrate enhanced binding of [³⁵S]GTPγS to membranes expressing constitutively actived receptors. Assays measuring [³⁵S]GTP-γS binding are shown to confirm constitutive activation of the mutated human serotonin 5-HT_{2C} receptor. The advantage of using [³⁵S]GTP-γS binding to measure constitutive activation is that: (a) it is generically applicable to all G protein- coupled receptors; (b) it is proximal at the membrane surface making it less likely to pick-up molecules which affect the intracellular cascade.

The assay utilizes the ability of G protein coupled receptors to stimulate [³⁵S]GTP-γS binding to membranes expressing the relevant receptors. The assay can, therefore, be used in the direct identification method to screen candidate compounds to known, orphan and constitutively activated G protein coupled receptors. The assay is generic and has application to drug discovery at all G protein coupled receptors.

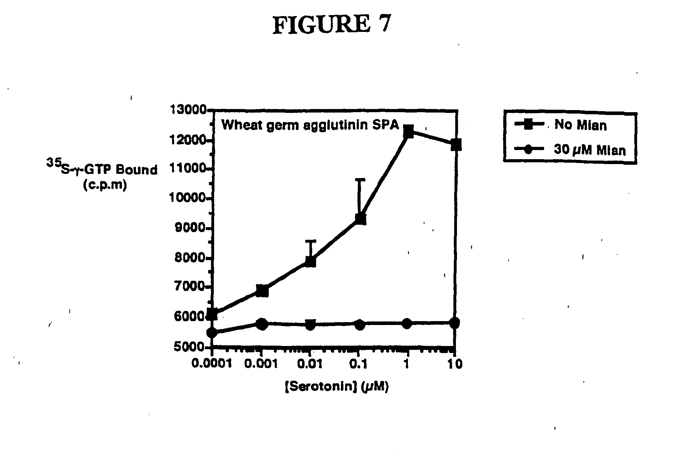

Figure 7 demonstrates the utility of a scintillation proximity assay to monitor binding of [³⁵S]GTP-γS to membranes expressing the native human 5-HT_{2C} receptor expressed in COS cells. Briefly, the assay is incubated in 20 mM HEPES pH 7.4 binding buffer with 0.3 nM [³⁵S]GTPγS and 12.5 µg membrane protein and 1 µM GDP for 30 min. Wheatgerm agglutinin beads (25 µl; Amersham) were then added and the mixture incubated for a further 30 min at room temperature. The tubes were then centrifuged @ 1500 X g for 5 min at room temperature and then counted in a scintillation counter. As can be seen in Figure 7, serotonin, which as the endogenous ligand activates the 5-HT_{2A} receptor, stimulated [³⁵S]GTPγS binding to the membranes in a concentration dependant manner. The stimulated binding was completely inhibited by 30 µM mianserin, a compound considered a classical 5-HT_{2A} antagonist, but also known to be an inverse agonist. Although this assay measures agonist-induced binding of [³⁵S]GTPγS to membranes and can be routinely used to measure constitutive activity of receptors. However, the present cost of wheatgerm agglutinin beads may be prohibitive.

A less costly but equally applicable alternative has been identified which also meets the needs of large scale screening. Flash plates and Wallac^{™} scintistrips may be utilized to format a high throughput [³⁵S]GTPγS binding assay. Furthermore, using this technique, the assay can be utilized to simultaneously monitor tritiated ligand binding to the receptor at the same time as monitoring the efficacy via [³⁵S]GTPγS binding. This is possible because the Wallac beta counter can switch energy windows to look at both tritium and ³⁵S labeled probes. This assay may also be used to detect other types of membrane activation events resulting in receptor activation. For example, the assay may be used to monitor ³²P phosphorylation of a variety of receptors (both G protein coupled and tyrosine kinase receptors). When the membranes are centrifuged to the bottom of the well, the bound [³⁵S]GTPγS or the ³²P-phosphorylated receptor will activate the scintillant which is coated of the wells. Scinti^{●} strips (Wallac) have been used to demonstrate this principle. In addition, the assay also has utility for measuring ligand binding to receptors using radioactively labeled ligands. In a similar manner, when the radiolabeled bound ligand is centrifuged to the bottom of the well, the scintistrip label comes into proximity with the radiolabeled ligand resulting in activation and detection.

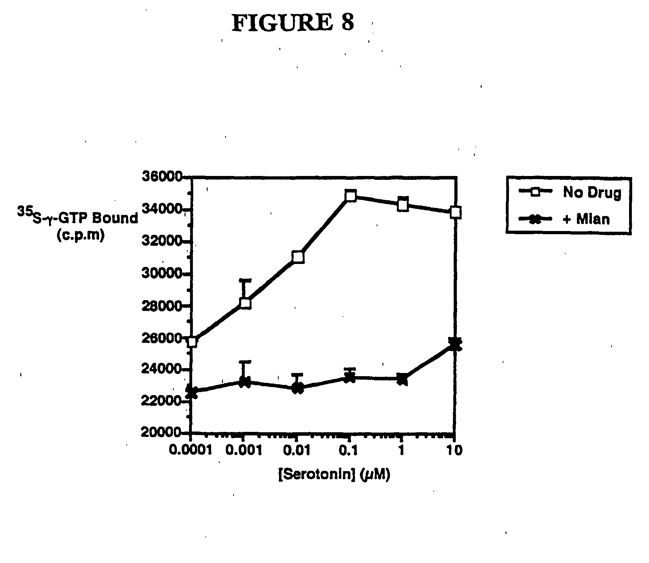

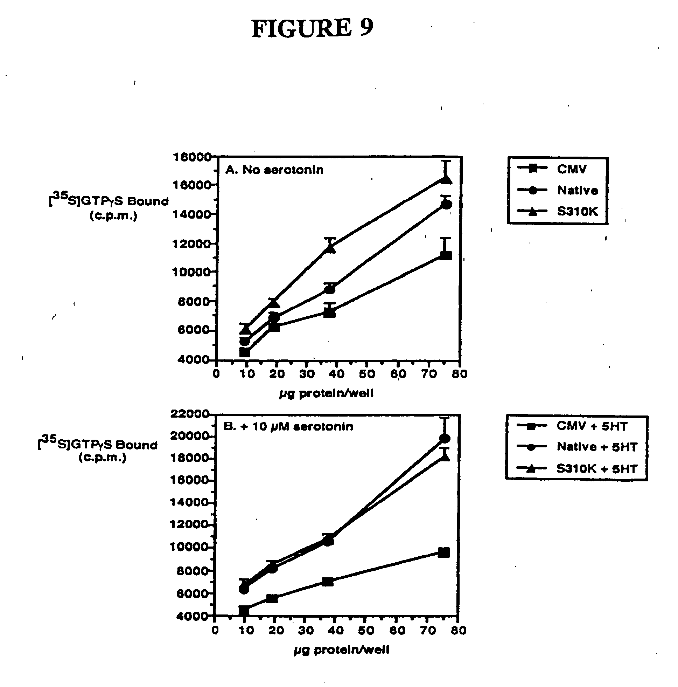

[³⁵S]GTPγS assay results parallel results obtained in traditional second messenger assays of receptors. As shown in Figure 8, serotonin stimulates binding of [³⁵S]GTPγS to the human 5-HT_{2C} receptor, while mianserin inhibits this response. Furthermore, mianserin acts as a partial inverse agonist and inhibits basal constitutive binding of [³⁵S]GTPγS to membranes expressing the native human 5-HT_{2C} receptor. As expected, there is no agonist response in the absence of GDP since there is no GDP present to exchange for [³⁵S]GTPγS (data not shown). Not only is this assay system valid for demonstrating the response of the native 5-HT_{2C} receptor, but it is also valid for measuring the constitutive activation of other receptors. Figure 9 demonstrates the enhanced binding of [³⁵S]GTPγS to membranes prepared from 293T cells expressing the control vector alone, the native human 5-HT_{2C} receptor or the S310K constitutively active mutant human 5-HT_{2C} receptor. The total protein concentration used in the assay affects the total amount of [³⁵S]GTPγS binding for each receptor. The c.p.m. differential between CMV transfected and the constitutively active mutant receptor increased from approximately 1000 c.p.m at 10 µg/well to approximately 6-8000 c.p.m. at 75 µg/well protein concentration, as can be seen in Figure 9. The S310K mutant receptor showed the highest level of constitutive activation followed by the wild type receptor which also showed enhanced [³⁵S]GTPγS binding above basal. This demonstration is consistent with the ability of the native human 5-HT_{2C} receptor to accumulate intracellular IP₃ in the absence of 5HT stimulation (Figure 5) and with published data claiming that the native 5-HT_{2C} receptor has a high natural basal activity. Thus, the S310K mutation of the human 5-HT_{2C} receptor clearly demonstrates that constitutive activity may be measured by proximal [³⁵S]GTPγS binding events at the membrane interface. Upon serotonin stimulation, both the endogenous and non-endogenous (S310K) receptors are stimulated to bind more [³⁵S]GTPγS.

### EXAMPLE 3

### Gal4 Reporter Assays In LVIP2.OZc Cells And Its Use In Identifying Active Receptors

To generate a β-galactosidase reporter containing multiple Gal4 binding sites, a Bgl II/HindIII fragment was removed from the somatostatin promoter-containing plasmid 1.4(5xGal)CAT [Leonard, J. et al (1992) Proc Natl Acad Sci USA 89:6247-6251] and cloned into pβgal-Basic (Promega). The Bgl II/ HindIII fragment contains a variant of the minimal somatostatin promoter (from -71 bp to +50 bp relative to the transcription start site) in which the core 4bp of the cAMP Response Element (-46 to -43) have been replaced with 5 copies of the recognition sequence for the yeast transcription factor Gal4. When this reporter is co-transfected with an expression plasmid encoding a Gal4-CREB fusion protein, it is highly responsive to agents that increase the cAMP signaling pathway.

VIP2.0Zc is a cell line that has been stably transfected with the reporter gene β-galactosidase under the control of a cAMP responsive VIP promoter (Konig et al. Molecular and Cellular Neurosciences 1991, 2, 331-337). The cell line was used here to indirectly measure the accumulation of intracellular cAMP. Approximately 2 million cells were plated in 6 cm plate the day before transfection. DNA (5 µg), for each receptor, was mixed with 2.5 ml serum-free DMEM containing 200 µg/ml DEAE dextran and 100 µM chloroquine, and added to a rinsed cell monolayer. After incubation for 90 min in the CO₂ incubator, the transfection medium was removed. The cells were washed with serum-free medium and supplemented with fresh complete medium. Twenty four hours after transfection, the cells were replated into 96-well plate at a density of 50 - 100 K per well and the β-galactosidase activity was assayed 48 to 72 hours after transfection. Compounds were tested after exposure to the cells overnight.

The assay buffer contains 100 mM sodium phosphate, 2 mM MgSO₄, 0.1 mM MnCl₂, pH 8.0. The cells were washed with PBS, and 25 µl /well of hypotonic lysis buffer consisting of 0.1 X assay buffer was added. Ten minutes later, 100 µl of assay buffer containing 0.5% Triton X-100 and 40 mM β-mercaptoethanol was added to each well and incubation at room temperature continued for 10 minutes. The substrate solution containing 5 mg/ml chlorophenol red-β-D-galactopyranoside (CPRG) in assay buffer was added at 25 µl/well and the plate was incubated at 37°C for 30 minutes before absorbance at 595 nm was measured with a plate reader.

### EXAMPLE 4

### Endogenous, Constitutively Activated Orphan Receptor, Homologues Thereof, Tissue Distribution, and Direct Identification of Inverse Agonists Thereto

The power of the methodologies disclosed herein is illustrated below using GPR3. GPR3 is a G protein coupled orphan receptor known to be constitutively activated in its endogenous form (Eggerickx, D. et al. (1995)).

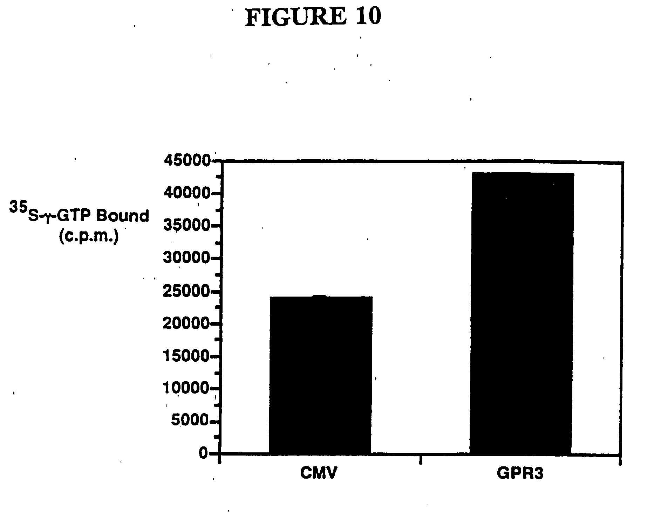

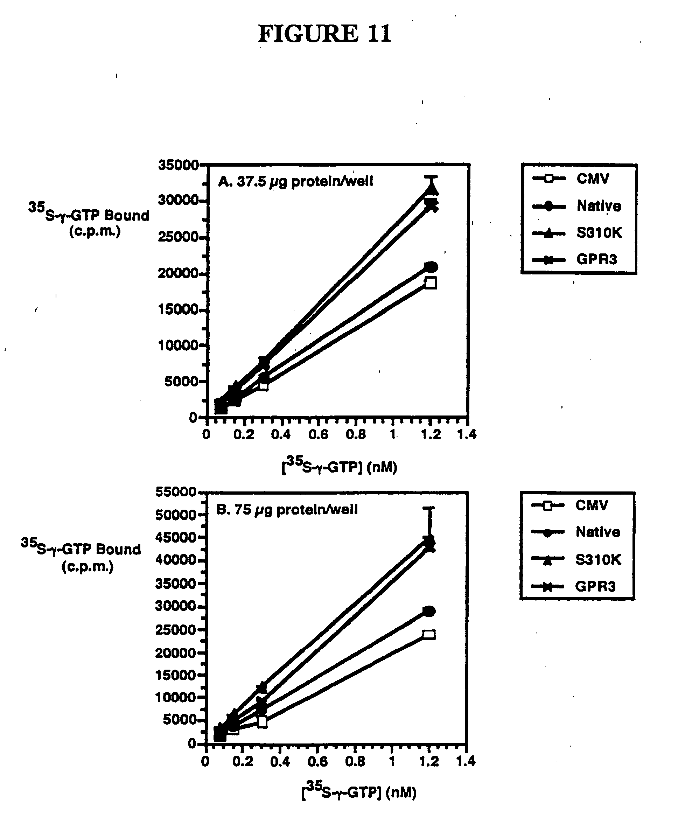

Referring to Figure 10, and using the [³⁵S]GTPγS binding assay system of Example 2, GPR3 receptor was determined to have increased activity as compared to control; this heightened activity is not the result of autocrine stimulation in that the data were obtained from membrane preparations, as opposed to whole cell preparations. Not only is GPR3 a constitutively activated receptor, but its level of activity is comparable to that of the mutated human 5-HT_{2A} receptor, as shown in Figure 11 using the [³⁵S]GTPγS binding assay system (75 µg/well of membrane protein [ie, 293T cells expressing GPR3] was incubated for 1 hr with 12 nM [³⁵S]GTPγS and 1 µM GDP).

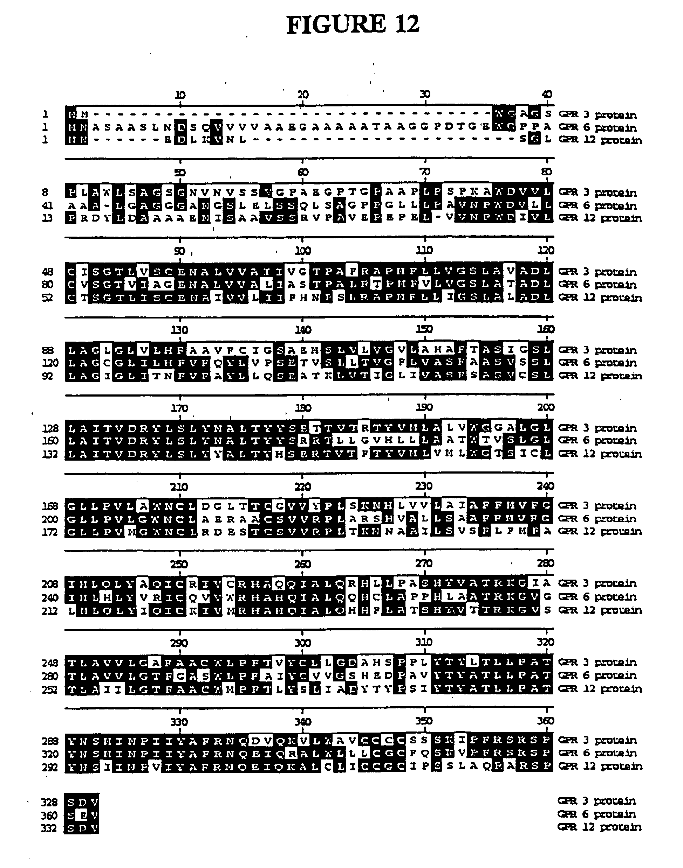

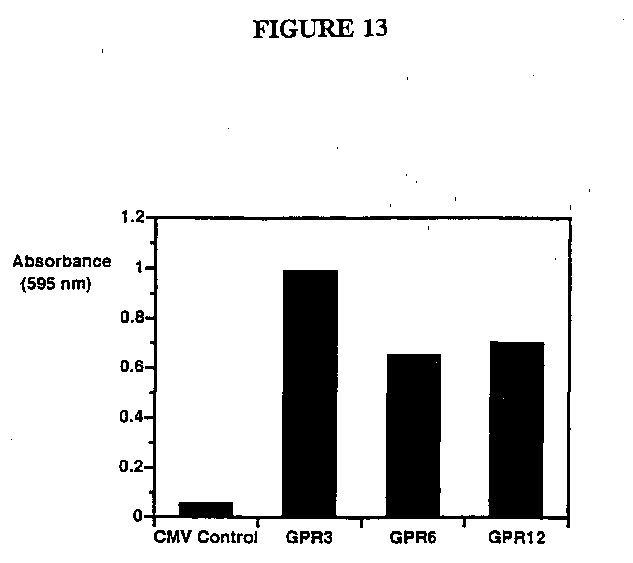

Following confirmation that GPR3 is a constitutively activated receptor, a first significant divergence from the "dogma" approach to drug identification was employed - this divergence is only possible as a result of the present invention. A homology search of the available G protein-coupled data banks (GeneBank), using the commercially available program, *DNA Star,* identified two highly homologous receptors, GPR6 and GPR12 (see Figure 12); both of these receptors are orphan receptors. While the sequence of these receptors was previously "known" (i.e., they were available on the databases), it was not known that these two receptors are constitutively activated in their endogenous forms (Figure 13). Furthermore, heretofore there would be no reason to search for such receptors for use in a drug discovery program in that the ligands therefore are not known. As such, the dogma approach to drug discovery would at best find the homology between GPR3, GPR6 and GPR12 of minor interest or, more likely, irrelevant.

Following discovery of two orphan homologues of GPR3, i.e., GPR6 and GPR12, a second significant divergence from the dogma approach to drug discovery was employed - again, this divergence is only possible as a result of the present invention. Tissue samples were examined for expression of these orphan receptors by comparative RT-PCR, using the following primers:
GPR3:
   5'-CTGGTCCTGCACTTTGCTGC-3'
   5'-AGCATCACATAGGTCCGTGTCAC-3'
   These primers amplify a 194bp fragment.
GPR6 :
   5'-ACCAGAAAGGGTGTGGGTACACTG-3'
   5'-GGAACGAAAGGGCACTTTGG-3'
   These primers amplify a 249bp fragment.
GPR12:
   5'-GCTGCCTCGGGATTATTTAG-3'
   5'-GCCTATTAGCAGGAACATGGGTG-3'
   These primers amplify a 220bp fragment.

These amplicons were designed to be non-overlapping, i.e., there is no sequence similarity between them, and to have similar Tm, such that each primer pair amplifies its respective target at the same optimal annealing temperature. This diminishes the chance that an amplicon from one primer pair will act as an annealing target for the other primers in the multiplex reaction, therefore reducing the chance of interference with other primer pairs.

Total RNA was extracted from tissue samples (human) using TRIzol^{™} Reagent (Gibco/BRL), following manufacturer instructions. cDNA was generated using 2mg total RNA and a First-Strand^{™} cDNA synthesis kit (Pharmacia). The cDNA samples were then diluted 1:3 in H₂O and comparative PCR was performed as described (Jensen, J. et al. (1996) J. Biol. Chem. 271:187490 in the presence of [³²P]dCTP. All reactions included the SP1-specific primers, which amplify a 300bp fragment, to serve as an internal control. Using the primers outlined above, under defined PCR conditions (1 cycle: 95°C, 5min; 23 cycles: 95°C, 30sec, 58°C, 30sec, 72°C, 1min; I cycle: 72°C, 10min) gave consistently reliable and quantitatively accurate results. It was further determined that the selected primer pairs did not interfere with each other when multiplexed. PCR products were visualized by denaturing gel electrophoresis (7M urea, 5% polyacrylamide (Long Ranger^{™} Solution, AT Biochemical, 0.6 XTBE) and subsequent autoradiography.

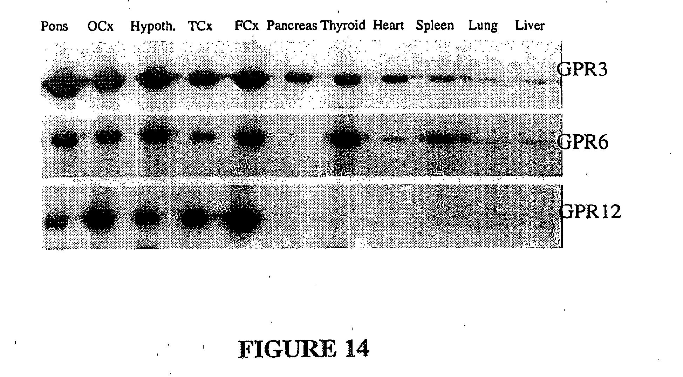

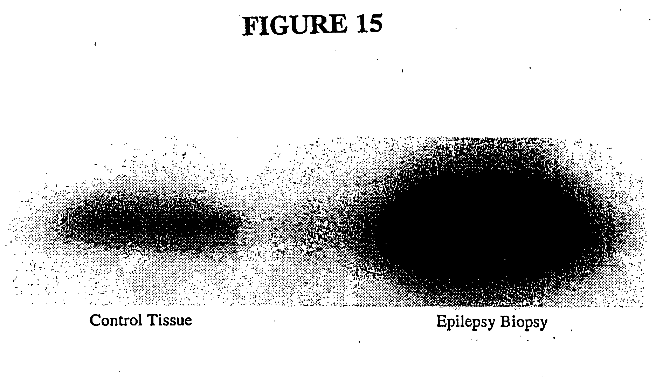

Figures 14A, 14B, and 14C show the distribution of GPR3, GPR6 and GPR12 across human tissues. This information allows for assessing disease states that are associated with such tissue, as well as determining specific regions within such tissue where such expression predominates, thus allowing for correlating such receptor expression with particular disease states. This, in turn, then allows for direct identification of compounds which impact such receptors, without the need to understand or know the endogenous ligand for such receptor. Further screening reveals that GPR3 is expressed in much higher levels in human epilepsy tissue samples (tissue source: temporal cortex), as compared with controls, as evidenced by RT-PCR analysis (Figure 15). Again, under the dogma approach, there would be no rational incentive to conduct such an examination in that, at best, until the present invention, such information would have been considered, perhaps, as interesting, albeit, irrelevant, data.

Thus, even without knowledge of the endogenous ligands to three endogenous constituitively actived orphan receptors, the present invention allows for, and provides a rational basis for screening of candidate compounds to directly identify, most preferably, inverse agonists to these orphan receptors.

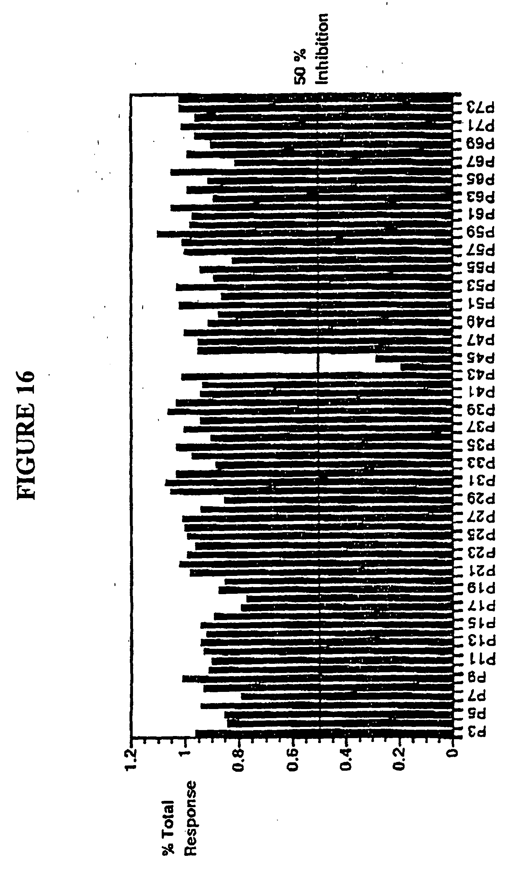

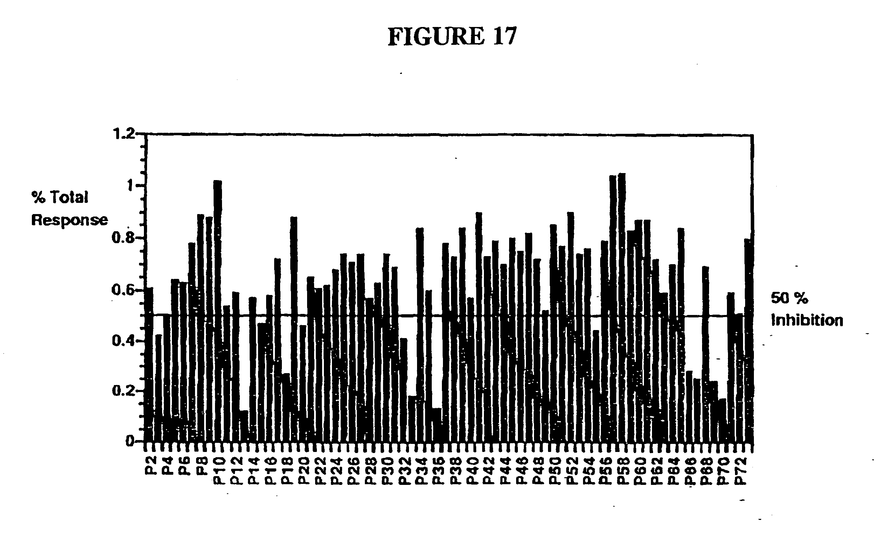

Using a β-galactosidase system (see Example 3, supra), 720 candidate compounds (Tripos, Inc.) were screened against the GPR6 receptor, and 74 candidate compounds were screened against the GPR3 receptor. These compounds were incubated on the cells overnight. Of these, two compounds were directly identified as being inverse agonists against the GPR3 receptor (P44 and P45, Figure 16), and 13 were directly identified as being inverse agonists against the GPR6 receptor (P3, P13, P15, P18, P20, P32, P33, P36, P55, P66, P67, P69, and P70, Figure 17).

The data support the position that the present invention effectively and efficiently allows for direct identification of inverse agonists against a receptor for which the endogenous ligand is unknown. Because of this technology, correlating the distribution of orphan receptors in specialized tissue and/or correlating the presence of such receptors with specified diseases allows for a rational approach to the development of a pharmaceutical composition(s) for such diseases.

### APPENDIX "A"

Examples of drugs and non-peptides which have been discovered by ligand-based receptor screens.

| Drug/non-peptide | Radiolabel For Screening | Reference | Targeted Receptor |
|---|---|---|---|
| Zantac | [³H]-Histamine | 1 | Histamine H2 |
| Tagamet | [³H]-Histamine | 1 | Histamine H2 |
| Seldane | [³H]-Histamine | 1 | Histamine H1 |
| Ventolin | [³H]-Noradrenaline | 2 | Adrenergic β2 |
| Atenolol/Tenermin | [³H]-Adrenaline | 2 | Adrenergic β1 |
| Propanolol/Inderal | [³H]-Noradrenaline | 2 | Adrenergic β1/β2 |
| Prazosin | [³H]-Adrenaline | 3 | Adrenergic α2B/2C |
| Isoproterenol/Isordil | [³H]-Noradrenaline | 2 | Adrenergic β1/β2 |
| Norepinephrine/ Levophed | [³H]-Adrenaline | 2 | Adrenergic α1/2,β1/2/3 |
| Imitrex | [³H]-5HT | 4 | Serotonin 5HT-1D |
| Buspar | [³H]-5-HT | 4 | Serotonin 5HT-1A |
| Nefazodone | [³H]-Ketanserin | 5 | Serotonin 5HT-2A |
| Thorazine | [³H]-Dopamine | 6 | Dopamine D2 |
| Clozapine | [³H]-Dopamine | 6 | Dopamine D2 |
| Codeine | [³H]-βendorphin | 7 | Opioid Receptors |
| Morphine | [³H]-βendorphin | 7 | Opioid Receptors |
| Methadone | [³H]-βendorphin | 7 | Opioid Receptors |
| RO-46-2005 | [¹²⁵I]-Endothelin | 8 | Endothelin |
| PD 156707 | | | |
| SB 209670 | | | |
| K-954 | [¹²⁵I]-Angiotensisn II | 9 | Angiotensin II |
| CP-154,526 | [¹²⁵I]-CRF | 10 | CRF |
| BIBP 3226 | [¹²⁵I]-NPY | 11 | NPY |
| SR-48968 | [¹²⁵I]-Neurokinin | 12 | Neurokinin |
| SR-49059 | [¹²⁵I]-Vasopressin | 13 | Vasopressin |
| L-366,509 | [¹²⁵I]-Oxytocin | 14 | Oxytocin |
| NK-1/NK-2 | [¹²⁵I]-Tackykinin | 15 | Tackykinin |
| LY303870 | [¹²⁵I]-Substance P | 16 | Substance P |
| HS-142-1 | [¹²⁵I]-ANP | 17 | ANP |
| L-365,269-CR 1505 | [¹²⁵I]-CCK | 18 | CCK |
| SR 48527 | [¹²⁵I]-Neurotensin | 19 | Neurotensin |
| WIN 64338 | [¹²⁵I]-Bradykinin | 20 | Bradykinin |

### References

- 1.: Hill, S. (1990). Distribution properties and functional characteristics of three classes of histamine receptor. Pharmacol. Review. 7, 1-51.
- 2.: Bylund, D. (1994). International union of pharmacology nomenclature of adrenoceptors. Pharmacol. Review., 46, 121-136.
- 3.: Hieble, J. (1995). International union of pharmacology. X. Recommendation for nomenclature of α1-adrenoceptors. Pharmacol. Review., 47, 267-270.
- 4.: Peroutka, S. (1995). Serotonin receptor subtypes. Their evolution and clinical relevance. CNS Drugs. 4, 19-28.
- 5.: Baxter, G. (1995). 5-HT2 receptors: a family re-united? Trends Pharmacol. Sci. 16, 105-110.
- 6.: Seeman, P. & Van Tol, H. (1994). Dopamine receptor pharmacology. Trends Pharmacol. Sci. 15, 264-270.
- 7.: Knapp, R. (1995). Molecular biology and pharmacology of cloned Opioid receptors. FASEB J. 9, 516-525.
- 8.: Reynolds, E.E. (1995).Pharmacological characterization of PD 156707, an orally active ETA receptor antagonist. J. Pharmacol. Exp. Their., 273:3, 1410-7. Gellai, M. et al (1995).Nonpeptide endothelin receptor antagonists. V: Prevention and reversal of acute renal failure in the rat by SB 209670. J. Pharmacol. Exp. Ther., 275:1, 200-6. Clozel, M. et al (1993).In vivo pharmacology of Ro 46-2005, the first synthetic nonpeptide endothelin receptor antagonist: implications for endothelin physiology. J. Cardiovasc. Pharmacol., 22 Suppl 8:, S377-9.
- 9.: Chen, T.S. et al (1993). Microbial hydroxylation and glucuronidation of the angiotensin II (AII) receptor antagonist MK 954. J. Antibiot. (Tokyo), 46:1, 131-4. Palkowitz, A.D. et al (1994). Structural evolution and pharmacology of a novel series of triacid angiotensin II receptor antagonists. J. Med. Chem., 37:26, 4508-21.
- 10.: Martone, R.L. et al (1996). Human CRF receptor chimeras: mapping of ligand binding determinants. Abstract 609.8. 26th meeting for the society of neuroscience, Washington, D.C., November 16-21, 1996.
- 11.: Sautel, M. et al (1996). Neuropeptide Y and the nonpeptide antagonist BIBP 3226 share an overlapping binding site at the human Y1 receptor. Mol. Pharmacol., 50:2, 285-92.
- 12.: Advenier, C. et al (1992). Effects on the isolated human bronchus of SR 48968, a potent and selective nonpeptide antagonist of the neurokinin A (NK2) receptors. Am. Rev. Respir. Dis., 146:5 Pt 1, 1177-81.
- 13.: Serradeil-Le Gal, C., et al (1993). Biochemical and pharmacological properties of SR 49059, a new, potent, nonpeptide antagonist of rat and human vasopressin V1a receptors. J. Clin. Invest., 92:1, 224-31.
- 14.: Pettibone, D.J. & Clineschmidt, B.V. (1993).Development and pharmacological assessment of novel peptide and nonpeptide oxytocin antagonists. Regul Pept, 29, 45:1- 2. Evans, B.E. et al (1992). Orally active, nonpeptide oxytocin antagonists. J. Med. Chem., 35:21, 3919-27.
- 15.: Kudlacz, E.M. et al (1996). In vitro and in vivo characterization of MDL 105,212A, a nonpeptide NK-1/NK-2 tachykinin receptor antagonist. J. Pharmacol. Exp. Ther., 277:2, 840-51.
- 16.: Gitter, B.D. et al (1995). Pharmacological characterization of LY303870: a novel, potent and selective nonpeptide substance P (neurokinin-1) receptor antagonist. J. Pharmacol. Exp. Ther., 275:2, 737-44.
- 17.: Imura, R. et al (1992). Inhibition by HS-142-1, a novel nonpeptide atrial natriuretic peptide' antagonist of microbial origin, of atrial natriuretic peptide-induced relaxation of isolated rabbit aorta through the blockade of guanylyl cyclase-linked receptors. Mol. Pharmacol., 42:6, 982-90. Oda, S. et al (1992). Pharmacological profile of HS-142-1, a novel nonpeptide atrial natriuretic peptide (ANP) antagonist of microbial origin. II. Restoration by HS-142-1 of ANP-induced inhibition of aldosterone production in adrenal glomerulosa cells. J. Pharmacol. Exp. Ther., 263:1, 241-5.
- 18.: Pendley, C.E. et al (1993). The gastrin/cholecystokinin-B receptor antagonist L- 365,260 reduces basal acid secretion and prevents gastrointestinal damage induced by aspirin, ethanol and cysteamine in the rat. J Pharmacol Exp Ther, 265:3, 1348-54. Rakovska, A. et al (1993). Effect of loxiglumide (CR 1505) on CCK-induced contractions and 3H-acetylcholine release from guinea-pig gallbladder. Neuropeptides, 25:5, 271-6. De Dios, I. & Manso, M.A. (1994). Effect of L-364,718 (CCK receptor antagonist) on exocrine pancreatic secretion of hydrocortisone-treated rats. Pancreas, 9:2, 212-8.
- 19.: Labbe-Jullie, C. (1994). Effect of the nonpeptide neurotensin antagonist, SR 48692, and two enantiomeric analogs, SR 48527 and SR 49711, on neurotensin binding and contractile responses in guinea pig ileum and colon. J. Pharmacol. Exp. Ther., 271:1, 267-76.
- 20.: Sawutz, D.G. et al (1995). Pharmacology and structure--activity relationships of the nonpeptide bradykinin receptor antagonist WIN 64338. Can. J. Physiol. Pharmacol., 73:7, 805-11.

### APPENDIX "B"

### PANLABS LIGAND RECEPTOR ASSAY LIST:

Drug screening labs such as Panlabs and Novascreen provide a service to check the specificity of drug leads at different receptor targets. The list of radio ligand receptor binding assays is set forth below. This information can be found at the web address of panlabs.

### Radioligand Binding Assays

Please note for this section:
Our standard procedure is to assay at the initial recommended
concentration in duplicate; if active (50%), concentration responses are carried out to determine IC50 ±SEM... (n=34 tubes). Other testing options are listed below.

1)Primary Screening and Quantitative Analysis (active compounds only):
   IC50 ±SEM, Ki, nH in Radioligand Binding Assays; IC50 ± SEM in Enzyme Assays, n=34 tubes per assay
2)Primary Screening and Semi-Quantitative Analysis: (10-5M and confirmation; 10-6, 10-7, 10-8M), n=10 tubes per assay
3)Three Point Primary Screen: (10-5, 10-7, 10-9M), n=6 tubes per assay
4)Primary Screen Only: (10-5 M), n=2 tubes per assay

$/Tube
Adenosine
Al (rat) $30
A2A (rat) $30
A3 (human) $50
Uptake Transporter (guinea pig) $40
Adrenergic
alphalA (human) $50
alphalB (rat) $30
alpha1, Non-Selective (rat) $30
alpha2A (human) $50
alpha2B (rat) $30
alpha2C (human) $50
alpha2, Non-Selective (rat) $30
beta1 (human) $50
beta2 (human) $50
beta3 (human) $50
beta, Non-Selective (rat) $30
Norepinephrine Transporter, (rat) $40
Angiotensin
AT1 (rabbit) $40
AT2 (rabbit) $40
Atrial Natriuretic Factor (guinea pig) $30
Bombesin (rat) $40
Bradykinin
B1 (human) $50
B2 (guinea pig) $40
Calcitonin (human) $40
Calcitonin Gene Related Peptide (rat) $40
Ca2+ Channel
Type L, Benzothiazepine (rat) $30
Type L, Dihydropyridine (rat) $30
Type L, Phenylalkylamine (rat) $30
Type N (rat) $40
Cannabinoid
CB1 (human) $50
CB2 (human) $50
Cholecystokinin
CCKA (human) $50
CCKB (human) $50
Choline Transporter (rat) $40
Dopamine
D1 (human) $50
D2S (human) $50
D3 (human) $50
D4.2 (human) $50
D4.4 (human) $50
D4.7 (human) $50
D5 (human) $50
Transporter (rat) $40
Endothelin
ETA (rat) $40
ETB (human) $50
Epidermal Growth Factor (human) $40
Estrogen (bovine) $40
GABA Transporter (rat) $40
GABAA
Agonist Site (rat) $30
Benzodiazepine, Central (rat) $30
Benzodiazepine, Peripheral (rat) $30
Chloride Channel, TBOB (rat) $40
GABAB (rat) $30
Galanin (rat) $40
Glucocorticoid (human) $40
Glutamate
AMPA (rat) $30
Kainate (rat) $30
NMDA, Agonist Site (rat) $30
NMDA, Glycine Site (rat) $30
NMDA, Phencyclidine Site (rat) $30
Glutamate, NMDA, Polyamine Site (rat) $30
Non-Selective (rat) $30
Glycine, Strychnine-Sensitive (rat) $30
Histamine
H1, Central (guinea pig) $30
H1, Peripheral (guinea pig) $30
H2 (guinea pig) $30
H3 (rat) $30
Imidazoline
I2, Central (rat) $30
I2, Peripheral (rat) $30
Inositol Trisphosphate, IP3 (rat) $40
Insulin (rat) $40
Interferon gamma (human) $50
Interleukin
IL-1alpha (mouse) $40
IL-2 (mouse) $75
IL-6 (human) $40
IL-8 (human) $40
Leukotriene
B4 (human) $40
D4 (guinea pig) $40
Melatonin, ML1 (chicken) $40
Monoamine Transporter (rabbit) $40
Muscarinic
M1 (human) $50
M2 (human) $50
M3 (human) $50
M4 (human) $50
M5 (human) $50
Non-Selective, Central (rat) $30
Oxotremorine-M (rat) $30
Neurokinin
NK1 (human) $50
NK2 (human) $50
Neuropeptide Y
Y1 (human) $40
Y2 (rabbit) $40
Neurotensin (mouse) $40
Nicotinic Acetylcholine, Central (rat) $30
Opiate
delta (guinea pig) $30
kappa (guinea pig) $30
mu (guinea pig) $30
Non-Selective (rat) $30
Phorbol Ester (mouse) $30
Platelet Activating Factor (rabbit) $30
Platelet-Derived Growth Factor (mouse) $50
Potassium Channel
[KA] (rat) $30
[KATP] (hamster) $30
[KV] (rat) $40
[SKCa] (rat) $40
Progesterone (bovine) $40
Purinergic P2X (rabbit) $30
Serotonin
5-HT1 (rat) $30
5-HT1A (human) $50
5-HT2 (rat) $30
5-HT3 (rabbit) $30
5-HT4 (guinea pig) $30
5-HT6 (human) $50
5-HT7 (human) $50
Transporter (rat) $40
Sigma
sigma 1 (guinea pig) $30
sigma 2 (rat) $30
Non-Selective (guinea pig) $30
Sodium Channel, Site 2 (rat) $40
Somatostatin (mouse) $40
Testosterone (rat) $40
Thromboxane A2 (rabbit) $30
Thyrotropin Releasing Hormone (rat) $40
Transforming Growth Factor-beta (mouse) $40
Tumor Necrosis Factor TNF-alpha (human) $40
Vasoactive Intestinal Peptide VIP1 (human) $50
Vasopressin V1 (rat) $40

### APPENDIX "C"

- 1.: Kosugi, S., et al (1993). Identification of thyroid-stimulating antibody-specific interaction sites in the N-terminal region of the thyrotropin receptor. Molecular Endocrinology, 7, 114-130.
- 2.: Parma, J., et al (1993). Somatic mutations in the thyrotropin receptor gene cause hyperfunctioning thyroid adenomas. Nature, 365, 649-651.
- 3.: Duprez, L., et al (1994). Germline mutations in the thyrotropin receptor gene cause non-autoimmune autosomal dominant hyperthyroidism. Nature Genetics, 7, 396-401.
- 4.: Matus-Leibovitch, N., et al (1995). Truncation of the thyrotropin-releasing hormone receptor carboxy tail causes constitutive activity and leads to impaired responsiveness in Xenopus oocytes and AtT20 cells. J. Biol. Chem., 270:3, 1041-1047.
- 5.: Kosugi, S., et al (1995). Characterization of heterogenous mutations causing constitutive activation of the luteinizing hormone receptor in familial male precocious puberty. Human Molecular Genetics, 4 (No.2), 183-188.
- 6.: Magnusson, Y., et al (1994). Autoimmunity in idiopathic dilated cardiomyopathy. Circulation, 89, 2760-2767.
- 7.: Fu, M., et al (1994). Functional autoimmune epitope on alpha1-adrenergic receptors in patients with malignant hypertension. Lancet, 344, 1660-1663.
- 8.: Samama, P., et al (1993a). A mutation-induced activation state of the B2-adrenergic receptor. J. Biol. Chem., 268:7, 4625-36.
- 9.: Kjelsberg, M.A., et al (1992). Constitutive activation of the alpha 1B-adrenergic receptor by all amino acid substitutions at a single site. J. Biol. Chem. 267, 1430-1433.
- 10.: Ren, Q., et al (1993). Constitutively active mutants of the alpha2-adrenergic receptor. J. Biol. Chem., 268, 16483-16487.
- 11.: Burstein, E.S., et al (1996). Constitutive activation of chimeric m2/m5 muscarinic receptors and delineation of G-protein coupling selectivity domains. Biochem Pharmacol, 51:4, 539-44. Burstein, E.S., et al (1996). Amino acid side chains that define muscarinic receptor/G-protein coupling. Studies of the third intracellular loop. J. Biol. Chem., 271:6, 2882-5.
- 12.: Hasegawa, H., et al (1996). Two isoforms of the prostaglandin E receptor EP3 subtype different in agonist-independent constitutive activity. J. Biol. Chem., 271:4, 1857-1860.
- 13.: Nanevicz, T., et al (1996). Thrombin receptor activating mutations. J. Biol Chem., 271, 702-706.
- 14.: Boone, C., et al (1993). Mutations that alter the third cytoplasmic loop of the a-factor receptor lead to a constitutive and hypersensitive phenotype. Proc. Natl. Acad. Sci. (USA), 90:21, 9921-5.
- 15.: Spiegel, A.M., et al (1995). Defects in G protein-coupled signal transduction in human diseases Ann. Rev. Physiol. 58, 143-170.
- 16.: Seeman, P. (1993). Dopamine D4 receptors elevated in schizophrenia. Nature, 365, 441-445.
- 17.: Mann, J., et al (1986). Increased serotonin2 and beta-adrenergic receptor binding in the frontal cortices of suicide victims. Arch. Gen. Psychiat. 43, 954-959. Casey, C., et al (1996). Constitutively active mutant 5-HT2A serotonin receptors: inverse agonist activity of classical 5HT2A antagonists. Soc.Neurosci. Abstracts # 699.10
- 18.: Barker, E.L., et al (1994). Constitutively active 5-hydroxytryptamine2C receptors reveal novel inverse agonist activity of receptor ligands. J. Biol. Chem., 269:16, 11687-11690.
- 19.: Maenhault, C., et al (1990). RCD8 codes for an adenosine A2 receptor with physiological constitutive activity. Biochem. Biophys. Res. Com., 173:3, 1169-1178.
- 20.: Parfitt, A.M., et al (1996). Hypercalcemia due to constitutive activity of the parathyroid hormone (PTH)/PTH-related peptide receptor: comparison with primary hyperparathyroidism. J. Clin. Endocr. Metab., 81, 3584-3588.
- 21.: Lavlie, R., et al (1996). The Ca(2+)-sensing receptor gene (PCAR1) mutation T151M in isolated autosomal dominant hypoparathyroidism. Hum Genet, 98:2, 129-33.
- 22.: Arvanitikis, L., et al (1997). Human herpesvirus KSHV encodes a constitutively active G-protein-coupled receptor linked to cell proliferation.
- 23.: Liu, J., et al (1996). Molecular mechanisms involved in muscarinic acetylcholine receptor-mediated G protein activation studied by insertion mutagenesis. J. Biol. Chem., 271:11, 6172-6178.
- 24.: Prezeau, L., et al (1996). Changes in the carboxy-terminal domain of metabotropic glutamate receptor 1 by alternate splicing generate receptors with differing agonist- independent activity. Mol. Pharmacol., 49, 422-429.
- 25.: Alla, S.A., et al (1996). Extracellular domains of the bradykinin B2 receptor involved in ligand binding and agonist sensing defined by anti-peptide antibodies. 271, 1748- 1755.
- 26.: Wang, Z., et al (1994). Constitutive µ opioid receptor activation as a regulatory mechanism underlying narcotic tolerance and dependance. Life Sciences, 54:22, 339- 350.
- 27.: Tiberi, M. & Caron, M.G. (1994). High agonist-independent activity is a distinguishing feature of the dopamine D1B receptor subtype. The J. Biol. Chem. 269:45. 27925- 27931.
- 28.: Robbins, L.S., et al (1993). Pigmentation phenotypes of variant extension locus alleles result from point mutations that alter MSH receptor function. Cell, 72, 827-834.
- 29.: Eggerick, D., et al (1995). Molecular cloning of an orphan G-protein-coupled receptor that constitutively activates adenylate cyclase. Biochem. J. 389, 837-843.
- 30.: Jakub/Eik, J., et al (1995). Constitutive activity of the M1-M4 subtypes of muscarinic receptors in transfeceted CHO cells and of muscarinic receptors in the heart cells revealed by negative antagonists. FEBS Lett, 377:2, 275-9.
- 31.: Burstein, E.S., et al (1995). Constitutive activation of muscarinic receptors by the G- protein Gq. FEBS Lett, 363:3, 261-3.
- 32.: Ter Lack, A., et al (1995). Modelling and mutation studies on the histamine H1- receptor agonist binding site reveal different binding modes for H1-agonisis: Asp116 (TM3) has a constitutive role in receptor stimulation. J. Computer-aided molecular design, 9, 319-330.

### APPENDIX "D"

### THREE AND SINGLE LETTER AMINO ACID ABBREVIATIONS

| | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HITIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

## Claims

1. An in vitro method for directly identifying a candidate compound as a compound that stimulates an orphan G-protein-coupled receptor, or a compound which acts to diminish the active state of the orphan G-protein-coupled receptor, wherein the orphan G protein-coupled receptor comprises a mutation in its amino acid-sequence so as to render it a non-endogenous constitutively activated orphan G protein-coupled receptor, said method comprising the steps of
(a) contacting said candidate compound with said non-endogenous constitutively activated orphan G protein-coupled receptor, wherein said orphan G protein-coupled receptor is expressed on a mammalian cell; and
(b) determining, by measurement of the ability of the compound to inhibit or stimulate receptor functionality, wherein said candidate compound is a compound that stimulates said orphan G protein-coupled receptor or a compound which acts to diminish the active state of said orphan G protein-coupled receptor,

2. The method of claim 1 wherein said mutation includes single amino acid mutations.

3. The method of claim 1 wherein said mutation is produced by using a mutational cassette.

4. The method of claim 1 wherein the compound is determined to be an inverse agonist to said receptor.

5. The method of claim 1 wherein the third intracellular loop of said orphan G protein-coupled receptor comprises the following sequences:
X1BBHyX2 wherein X1 is an amino acid; B is a basic amino acid; Hy is a hydrophobic amino acid, and X2 is an amino acid.

6. The method of claim 5 wherein X1 is glycine.

7. The method of clam 5 wherein X1 is alanine.

8. The method of claim 5 wherein X1 is lysine.

9. The method of claim 5 wherein Hy is alanine

10. The method of claim 5 wherein X2 is lysine

11. The method of claim 5 wherein X2 is arginine.

12. The method of claim 5 wherein X2 is glutamic acid

13. The method of claim 1 wherein the second intracellular loop of said orphan G protein-coupled receptor comprises the following sequences:
XRY wherein X can be any amino acid other than D.

14. The method of claim 5 wherein said sequence X1BBHyX2 is an endogenous sequence.

15. The method of claim 5 wherein said sequence X1BBHyX2 is a non-endogenous sequence.

16. The method of claim 13 wherein the sequence XRY is an endogenous sequence.

17. The method of claim 13 wherein the sequence XRY is a non-endogenous sequence.

18. A method according to any one of the preceding claims wherein the ability of the compound to inhibit or stimulate receptor functionality is detected by measuring the change in cAMP levels when said candidate compound is contacted with said constitutively activated orphan G protein-coupled receptor.

19. A method according to any one of the preceding claims wherein the ability of the compound to inhibit or stimulate receptor functionality is detected by measurement of [³⁵S]GTPγS binding.

## Patentansprüche

1. In-vitro-Verfahren zum direkten Identifizieren einer Kandidatenverbindung als Verbindung, die einen G-Protein-gebundenen Waisenrezeptor stimuliert, oder als Verbindung, die eine Abschwächung des aktiven Zustands eines G-Protein-gebundenen Waisenrezeptors bewirkt, wobei der G-Protein-gebundene Waisenrezeptor eine Mutation in seiner Aminosäuresequenz umfasst, die ihn zu einem nichtendogenen, konstitutiv aktivierten G-Protein-gebundenen Waisenrezeptor macht, wobei das Verfahren folgende Schritte umfasst:
(a) In-Kontakt-Bringen der Kandidatenverbindung mit dem nichtendogenen, konstitutiv aktivierten G-Protein-gebundenen Waisenrezeptor, wobei der G-Protein-gebundene Waisenrezeptor auf einer Säugetierzelle exprimiert wird; und
(b) das Bestimmen der Fähigkeit der Verbindung, die Rezeptorfunktionalität zu hemmen oder zu stimulieren, durch Messungen, wobei die Kandidatenverbindung eine Verbindung ist, die den G-Protein-gebundenen Waisenrezeptor stimuliert, oder eine Verbindung, die eine Abschwächung des aktiven Zustands des G-Protein-gebundenen Waisenrezeptors bewirkt.

2. Verfahren nach Anspruch 1, wobei die Mutation einzelne Aminosäuremutationen umfasst.

3. Verfahren nach Anspruch 1, wobei die Mutation unter Verwendung einer Mutationskassette erzeugt wird.

4. Verfahren nach Anspruch 1, wobei die Verbindung, die bestimmt werden soll, ein inverser Agonist für den Rezeptor ist.

5. Verfahren nach Anspruch 1, wobei die dritte intrazelluläre Schleife des G-Protein-gebundenen Waisenrezeptors folgende Sequenzen umfasst:
X1BBHyX2, worin X1 eine Aminosäure ist, B eine basische Aminosäure ist, Hy eine hydrophobe Aminosäure ist und X2 eine Aminosäure ist.

6. Verfahren nach Anspruch 5, wobei X1 Glycin ist.

7. Verfahren nach Anspruch 5, wobei X1 Alanin ist.

8. Verfahren nach Anspruch 5, wobei X1 Lysin ist.

9. Verfahren nach Anspruch 5, wobei Hy Alanin ist.

10. Verfahren nach Anspruch 5, wobei X2 Lysin ist.

11. Verfahren nach Anspruch 5, wobei X2 Arginin ist.

12. Verfahren nach Anspruch 5, wobei X2 Glutaminsäure ist.

13. Verfahren nach Anspruch 1, wobei die zweite intrazelluläre Schleife des G-Protein-gebundenen Waisenrezeptors folgende Sequenzen umfasst:
XRY, worin X eine beliebige Aminosäure mit Ausnahme von D ist.

14. Verfahren nach Anspruch 5, wobei die Sequenz X1BBHyX2 eine endogene Sequenz ist.

15. Verfahren nach Anspruch 5, wobei die Sequenz X1BBHyX2 eine nichtendogene Sequenz ist.

16. Verfahren nach Anspruch 13, wobei die Sequenz XRY eine endogene Sequenz ist.

17. Verfahren nach Anspruch 13, wobei die Sequenz XRY eine nichtendogene Sequenz ist.

18. Verfahren nach einem der vorangegangenen Ansprüche, wobei die Fähigkeit der Verbindung, die Rezeptorfunktionalität zu hemmen oder zu stimulieren, anhand der Messung der Veränderung der cAMP-Spiegel detektiert wird, wenn die Kandidatenverbindung mit dem konstitutiv aktivierten, G-Protein-gebundenen Waisenrezeptor in Kontakt gebracht wird.

19. Verfahren nach einem der vorangegangenen Ansprüche, wobei die Fähigkeit der Verbindung, die Rezeptorfunktionalität zu hemmen oder zu stimulieren, anhand der Messung der [³⁵S]GTPγS-Bindung detektiert wird.

## Revendications

1. Procédé in vitro pour identifier directement un composé candidat comme un composé qui stimule un récepteur orphelin couplé à la protéine-G, ou un composé qui agit pour diminuer l'état actif du récepteur orphelin couplé à la protéine-G, où le récepteur orphelin couplé à la protéine-G comprend une mutation dans sa séquence des acides aminés pour qu'il devienne un récepteur orphelin couplé à la protéine-G non-endogène constitutivement activé, ledit procédé comprenant les étapes de
(a) mettre en contact ledit composé candidat avec ledit récepteur orphelin couplé à la protéine G non-endogène constitutivement activé, où ledit récepteur orphelin couplé à la protéine G est exprimé sur une cellule de mammifère; et
(b) déterminer, par la mesure de l'aptitude du composé à inhiber ou stimuler la fonctionnalité du récepteur, où ledit composé candidat est un composé qui stimule ledit récepteur orphelin couplé à la protéine G ou un composé qui agit pour diminuer l'état actif dudit récepteur orphelin couplé à la protéine G.

2. Procédé selon la revendication 1, où ladite mutation inclut des mutations uniques des acides aminés.

3. Procédé selon la revendication 1, où ladite mutation est produite en utilisant une cassette mutationnelle.

4. Procédé selon la revendication 1, où le composé est déterminé comme étant un agoniste inverse audit récepteur.

5. Procédé selon la revendication 1, où la troisième boucle intracellulaire dudit récepteur orphelin couplé à la protéine G comprend les séquences suivantes:
X1BBHyX2 où X1 est un acide aminé; B est un acide aminé basique; Hy est un acide aminé hydrophobe et X2 est un acide aminé.

6. Procédé selon la revendication 5, où X1 est glycine.

7. Procédé selon la revendication 5, où X1 est alanine.

8. Procédé selon la revendication 5, où X1 est lysine.

9. Procédé selon la revendication 5, où Hy est alanine.

10. Procédé selon la revendication 5, où X2 est lysine.

11. Procédé selon la revendication 5, où X2 est arginine.

12. Procédé selon la revendication 5, où X2 est l'acide glutamique.

13. Procédé selon la revendication 1, où la deuxième boucle intracellulaire dudit récepteur orphelin couplé à la protéine G comprend les séquences suivantes: XRY où X peut être n'importe quel acide aminé autre que D.

14. Procédé selon la revendication 5, où ladite séquence X1BBHyX2 est une séquence endogène.

15. Procédé selon la revendication 5, où ladite séquence X1BBHyX2 est une séquence non-endogène.

16. Procédé selon la revendication 13, où la séquence XRY est une séquence endogène.

17. Procédé selon la revendication 13, où la séquence XRY est une séquence non-endogène.

18. Procédé selon l'une quelconque des revendications précédentes, où l'aptitude du composé à inhiber ou stimuler la fonctionnalité du récepteur est détectée par la mesure du changement dans les niveaux de AMPc lorsque le composé candidat est mis en contact avec ledit récepteur orphelin couplé à la protéine G constitutivement activé.

19. Procédé selon l'une quelconque des revendications précédentes, où l'aptitude du composé à inhiber ou stimuler la fonctionnalité du récepteur est détectée par la mesure de la fixation [³⁵S] GTPγS.
